# EUROPÄISCHE PATENTSCHRIFT

(11) **EP 2 515 796 B1**
(45) Veröffentlichungstag und Bekanntmachung des Hinweises auf die Patenterteilung: **30.09.2020**
(21) Anmeldenummer: 10801139.6
(22) Anmeldetag: 22.12.2010
(51) Int. Cl.: A61F 2/06, A61F 2/90

(54) **STENT MIT KLAPPEN**
STENT WITH FLAPS
STENT COMPRENANT DES VOLETS

(30) Priorität: 23.12.2009 DE 102009060228
(43) Veröffentlichungstag der Anmeldung: 31.10.2012
(73) Patentinhaber: Acandis GmbH, 75177 Pforzheim (DE)
(72) Erfinder: CATTANEO, Giorgio, 76199 Karlsruhe (DE)
(74) Vertreter: Kilchert, Jochen
(86) Internationale Anmeldenummer: PCT/EP2010/007888
(87) Internationale Veröffentlichungsnummer: WO 2011/076408

(56) Entgegenhaltungen:
- WO-A1-2008/057568
- WO-A1-2012/007145
- WO-A2-2007/117645
- US-A1- 2006 259 131
- US-A1- 2010 106 240

## Beschreibung

Die Erfindung betrifft eine medizinische Vorrichtung, vorzugsweise einen Stent oder Flow-Diverter bzw. allgemein eine Gefäßstütze. Derartige medizinische Vorrichtungen sind allgemein bekannt.

Medizinische Vorrichtungen der eingangs genannten Art werden üblicherweise in Blutgefäßen oder anderen Körperhohlräumen eingesetzt. Weitere Hohlräume, die mit derartigen medizinischen Vorrichtungen behandelbar sind, sind beispielsweise die Speiseröhre (Ösophagus), der Darm, der Gallengang, die Luftröhre, der Harnleiter oder dergleichen. Häufig werden derartige medizinische Vorrichtungen zur Behandlung von Stenosen, also Gefäßverengungen der Blutgefäße, insbesondere der Herzkranzgefäße, der größeren Arterien und/oder der zerebralen Gefäße verwendet. Eine weitere Einsatzmöglichkeit für die Verwendung derartiger bekannter medizinischer Vorrichtungen ist die Behandlung von Aneurysmen, also Gefäßaussackungen. Dabei bildet ein Blutgefäß eine lokale Ausweitung, wodurch die Gefäßwand in diesem Bereich stark gedehnt und beansprucht wird. Es besteht das Risiko, dass die gedehnte Gefäßwand reißt, wodurch es zu einer Blutung kommen kann.

Bekannte Gefäßstützen weisen üblicherweise eine Gitterstruktur auf, die zum Zuführen der Gefäßstütze in ein Blutgefäß auf einen kleineren Querschnittsdurchmesser komprimiert werden können. Im Blutgefäß weitet sich die Gefäßstütze auf bzw. expandiert die Gefäßstütze und übt auf die Gefäßwand eine radiale Kraft aus. Auf diese Weise können Gefäßverengungen aufgeweitet werden. Die Expansion der Gefäßstütze kann durch Aufweiten eines Ballons erfolgen, auf den die Gefäßstütze aufgebracht ist. Dazu wird der Ballon über einen Katheter mit einer Flüssigkeit gefüllt. Alternativ kann sich die Gitterstruktur der Gefäßstütze selbsttätig aufweiten. Derartige Gefäßstützen weisen üblicherweise ein Formgedächtnismaterial auf, das bei Körpertemperatur die zuvor festgelegte Form einnimmt.

Die Gitterstruktur der bekannten Gefäßstützen weist Zellen bzw. Maschen auf, deren Größe je nach Einsatzzweck variiert. Zur Abdeckung von Aneurysmen werden beispielsweise Gefäßstützen eingesetzt, deren Gitterstruktur eine relativ geringe Maschen- bzw. Zellgröße aufweist. Dadurch wird erreicht, dass die Blutströmung innerhalb des Aneurysmas gestoppt oder zumindest reduziert wird, so dass eine weitere Belastung der Gefäßwand innerhalb des Aneurysmas vermieden wird. Eine alternative Behandlungsmethode von Aneurysmen besteht darin, einzelne dünne Drahtelemente in das Aneurysma einzuführen, die sich im Aneurysma chaotisch kräuseln und somit den Blutfluss bzw. die Blutströmung innerhalb des Aneurysmas blockieren. Bei der Verwendung derartiger Drahtelemente, so genannter Coils, besteht die Gefahr, dass die Coils zumindest abschnittsweise aus dem Aneurysma in das Blutgefäß vorstehen und die Blutströmung innerhalb des Blutgefäßes beeinflussen, so dass sich Gerinnsel, insbesondere Thromben, innerhalb des Blutgefäßes bilden können. Die Coils können auch selbst aus dem Aneurysma herausgeschwemmt werden und in stromabwärts gelegenen Blutgefäßen einen Gefäßverschluss bewirken. Um dies zu vermeiden, wird das Blutgefäß zunächst mit einer Gefäßstütze im Bereich des Aneurysmas stabilisiert. Durch die Maschen bzw. Zellen der Gefäßstütze werden anschließend Coils in das Aneurysma eingeführt. Dabei ist die Wahl der Maschengröße der Gefäßstütze entscheidend. Größere Maschen erleichtern zwar das Einführen der Coils in das Aneurysma, ermöglichen aber gleichzeitig das Vorstehen einzelner Coilabschnitte in das Blutgefäß. Überdies lassen die größeren Maschen weiterhin einen Blutstrom innerhalb des Aneurysmas zu. Umgekehrt erschweren kleinere Maschen der Gefäßstütze die Zuführung von Coils in das Aneurysma.

Bei der Behandlung von Stenosen, insbesondere Stenosen, die eine vulnerable Plaque umfassen, weisen bekannte Gefäßstützen ebenfalls Nachteile auf. Eine vulnerable Plaque ist eine Ansammlung von Fettpartikeln und Körperzellen innerhalb eines Blutgefäßes, die durch eine dünne Gefäßinnenhaut bedeckt ist. Durch die vulnerable Plaque wird der Blutgefäßquerschnitt verengt, so dass sich die Stenose bildet. Bei der Aufweitung einer derartigen Stenose durch eine Gefäßstütze mit einer Gitterstruktur besteht die Gefahr, dass die Stege der Gitterstruktur die Gefäßinnenhaut verletzen und somit Partikel in die Blutströmung freigesetzt werden. Die freigesetzten Partikel können einen Gefäßverschluss in stromabwärts gelegenen Blutgefäßen bewirken.

Weitere bekannte Gefäßstützen umfassen eine flexible Folie, die die Gitterstruktur vollständig überspannt. Bei der Behandlung von Stenosen aus vulnerablen Plaques verhindert die Folie, dass sich ablösende Partikel in die Blutbahn bewegen und fortgeschwemmt werden. Häufig treten Stenosen im Bereich von Gefäßverzweigungen, beispielsweise im Bereich der Mündung der Vertebralarterie in die Basilararterie oder bei der Aufteilung der gemeinsamen Halsschlagader (A. carotis communis) in die innere Halsschlagader (A. carotis interna) und die äußere Halsschlagader (A. carotis externa) auf. Die Verwendung von Gefäßstützen mit einer geschlossenen Folie ist in derartigen Bereichen nicht möglich, da die Blutströmung in das abzweigende Blutgefäß unterbrochen werden würde. Gefäßstützen ohne eine Folie weisen hingegen die vorstehend genannten Nachteile bezüglich der der Partikelablösung auf.

Die Verwendung von folienbespannten Gefäßstützen zur Behandlung von Aneurysmen weist im Wesentlichen dieselben Nachteile auf, wie vorstehend bezüglich der bekannten Flow-Diverter beschrieben, die eine relativ kleine Maschengröße aufweisen. Sowohl bei den bekannten Flow-Divertern, als auch bei folienbespannten Gefäßstützen ist es nicht möglich, einen Katheter zur Zufuhr von Coils in das Aneurysma zu führen.

WO 2008/057568 A1 offenbart einen Stent mit einer flexiblen Abdeckung (Graft), die den Stent vollständig bedeckt. In der Abdeckung sind Klappen ausgebildet.

WO 2010/048177 A2 zeigt einen Stent, in dem ein großflächiges Fenster ausgebildet ist, das zum Einbringen von Coils einen Zugang zu einem Aneurysma ermöglichen soll.

WO 2007/117645 A2 offenbart ein Verschlusssystem für Aneurysmen. Das Verschlusssystem umfasst einen rohrförmigen Stent mit mehreren Streben, welche großflächige Zellen begrenzen. In den Zellen sind mehrere Klappen angeordnet, die in eine Offen- und Schließstellung überführbar sind. Die Klappen sind nicht direkt, sondern über eine zusätzliche Seitenstrebe mit der Stentstruktur verbunden. Das hat den Nachteil, dass die Klappe die in der Stentstruktur ausgebildete Öffnung nur über eine vergleichsweise kleine Fläche abgedeckt werden kann. Außerdem erscheint die Seitenstrebe wenig stabil, was im Hinblick auf die im pulsierenden Blutstrom auftretenden Druckschwankungen als nachteilig angesehen wird.

Die Aufgabe der Erfindung besteht darin, eine medizinische Vorrichtung anzugeben, die einerseits eine zuverlässige Stützung eines Körperhohlraums und andererseits eine seitliche Fluidströmung ermöglicht. Insbesondere besteht die Aufgabe der Erfindung darin, eine medizinische Vorrichtung anzugeben, mit der eine Fluidströmung innerhalb eines Körperhohlraums gezielt zeitlich steuerbar ist. Die Erfindung soll vorzugsweise eine medizinische Vorrichtung bereitstellen, die eine Stenose und/oder ein Aneurysma zuverlässig abdeckt und gleichzeitig eine seitliche Fluidströmung im Bereich bzw. in der Nachbarschaft der Stenose bzw. des Aneurysmas ermöglicht.

Ferner liegt der Erfindung die Aufgabe zu Grunde, eine medizinische Vorrichtung anzugeben, die eine axiale Fluidströmung entgegen der Hauptströmungsrichtung des innerhalb des Körperhohlraums geführten Fluids verhindert und eine Fluidströmung in Hauptströmungsrichtung des Körperfluids ermöglicht.

Erfindungsgemäß wird diese Aufgabe im Hinblick auf die seitliche Fluidströmung durch den Gegenstand des Patentanspruchs 1 und im Hinblick auf die axiale Fluidströmung durch den Gegenstand des Patentanspruchs 2 gelöst.

Die Erfindung beruht auf dem Gedanken, eine medizinische Vorrichtung mit einer rohrförmigen Wandung aus Stegen, die Zellen begrenzen, und einer flexiblen Membran anzugeben, die wenigstens eine Klappe bildet. Die Klappe weist ein erstes Ende auf, das mit wenigstens einem ersten Steg einer Zelle verbunden ist. Ferner weist die Klappe ein freies zweites Ende auf, das dem ersten Ende in Längsrichtung der Klappe gegenüber angeordnet ist. Die Klappe ist in eine Schließstellung und in eine Offenstellung überführbar. In der Schließstellung erstreckt sich die Klappe entlang der rohrförmigen Wandung und verschließt die Zelle zumindest teilweise. In der Offenstellung ist die Klappe relativ zur Wandung radial ausgelenkt, um die Zelle ventilartig freizugeben.

Die Erfindung basiert auf der Idee, die Zellen der rohrförmigen Wandung mit einer flexiblen Membran zu bedecken, die klappenartig ausgebildet ist. Die klappenartige Membran bzw. die Klappe ist radial auslenkbar, um die Zelle in der Schließstellung zu bedecken und in der Offenstellung die Zellöffnung freizugeben. Die Klappe weist somit eine Ventilfunktion auf.

Die Klappe nimmt ihre Schließstellung bzw. Offenstellung erfindungsgemäß selbsttätig ein, und zwar in Folge eines Druckgradienten oder einer Fluidströmung. Der Druckgradient bzw. die Fluidströmung kann zwischen einem inneren Hohlkanal, der sich in axialer Richtung innerhalb der rohrförmigen Wandung erstreckt, und einen Außenraum ausgebildet sein, der sich außerhalb, insbesondere radial außerhalb, der rohrförmigen Wandung ausdehnt.

Es ist auch möglich, dass die Schließstellung bzw. Offenstellung der Klappe durch manuelle Betätigung einstellbar ist. Beispielsweise kann die Klappe durch äußere Krafteinwirkung, vorzugsweise durch die Vorschubkraft eines Katheters, geöffnet werden. Insgesamt ermöglicht die Klappe eine zuverlässige Abdeckung der Zelle in der Schließstellung, um beispielsweise eine Blutströmung in ein Aneurysma zu blockieren. Gleichzeitig ist die Klappe in die Offenstellung überführbar, so dass beispielsweise Coils in ein Aneurysma eingebracht werden können. Sobald das Aneurysma mit einer ausreichenden Anzahl von Coils gefüllt und der Zufuhrkatheter zurückgezogen ist, verschließt die Klappe die Zelle bzw. Zellöffnung selbsttätig. Somit wird zusätzlich verhindert, dass Coils aus dem Aneurysma in die Blutbahn gelangen.

Allgemein ist vorgesehen, dass die Klappe in der Schließstellung vollständig auf der rohrförmigen Wandung aufliegt. Die Klappe erstreckt sich also im Wesentlichen parallel entlang der rohrförmigen Wandung und zwar im Wesentlichen in vollständiger Anlage mit der rohrförmigen Wandung. Alternativ kann die Klappe im Rahmen der Anmeldung auch dann in der Schließstellung angeordnet sein bzw. die Schließstellung aufweisen, wenn die Klappe geringfügig gegenüber der rohrförmigen Wandung ausgelenkt ist. Das trifft insbesondere im Herstellzustand bzw. Ruhezustand der medizinischen Vorrichtung zu. Im Herstellzustand der medizinischen Vorrichtung kann die Klappe also geringfügig nach radial außen oder nach radial innen ausgelenkt sein, wobei vorteilhafterweise eine signifikante Öffnung der Zelle vermieden ist. Diese in der Schließstellung vorliegende Grundauslenkung kann der Klappe beispielsweise durch eine Wärmebehandlung aufgeprägt sein. Die Grundauslenkung kann derart angepasst sein, dass die Klappe im implantierten Zustand, beispielsweise durch Anlage an eine Gefäßwand, parallel und bündig auf der rohrförmigen Wandung aufliegend anordenbar ist. Die bündige Anlage der Klappe an die rohrförmige Wandung im implantierten Zustand kann auch durch den Einfluss einer Fluidströmung erreicht werden, die auf die Klappe wirkt. Im Allgemeinen ist die Auslenkung zur Herstellung der Offenstellung der Klappe wesentlich größer als die Grundauslenkung in der Schließstellung der Klappe. Die Ventilfunktion der Klappe kommt bei der Überführung der Klappe von der Schließstellung in die Offenstellung und umgekehrt zum Tragen.

Mit der erfindungsgemäßen medizinischen Vorrichtung sind insbesondere Aneurysmen zuverlässig abdeckbar, wobei gleichzeitig die Zufuhr von Coils in das Aneurysma ermöglicht ist. Aufgrund der Klappen, die die Zellen bedecken, können die Abstände der Stege der rohrförmigen Wandung, also die Zellengröße vergleichsweise groß gewählt werden, um einen Katheter durch die rohrförmige Wandung zu führen. Das hat den Vorteil, dass die medizinische Vorrichtung eine verbesserte Crimpbarkeit, also einen relativ kleinen Querschnittsdurchmesser im komprimierten Zustand aufweist, da die Anzahl der Stege in Umfangsrichtung der rohrförmigen Wandung reduziert ist. Gleichzeitig wird die Flexibilität der Vorrichtung durch die geringe Steganzahl erhöht.

Besonders vorteilhaft ist die erfindungsgemäße medizinische Vorrichtung bei Aneurysmen, die im Bereich von Gefäßverzweigungen ausgebildet sind. Dabei ermöglicht die flexible Klappe einerseits eine zuverlässige Abdeckung der Aneurysmen, so dass es innerhalb des Aneurysmas zu einer vorteilhaften Embolisierung kommt, so dass der Blutfluss und somit die Belastungen auf die Gefäßwand innerhalb des Aneurysmas reduziert werden. Gleichzeitig kann die Klappe oder eine weitere Klappe, durch die Blutströmung, die im Bereich der Gefäßverzweigung in das seitliche Gefäß, in den das Aneurysma ausgebildet ist, von der Schließstellung in die Offenstellung überführbar sein, so dass sich eine natürliche Strömungspassage bildet, in der das Blut bzw. allgemein das Körperfluid an dem Aneurysma vorbei in das seitliche Gefäß strömen kann.

Die Klappe ist einseitig, nämlich am ersten Ende, mit einem Steg der rohrförmigen Wandung verbunden. Das zweite Ende ist frei bzw. lose angeordnet. Das zweite Ende liegt dem ersten Ende in Längsrichtung der Klappe gegenüber. Das freie zweite Ende ist also gegenüber dem ersten Ende beweglich. Das ermöglicht bei entsprechender Positionierung der medizinischen Vorrichtung im Bereich einer Gefäßverzweigung eine gezielte Beeinflussung der Fluidströmung innerhalb des Körperhohlgefäßes. In der Offenstellung ist die Klappe gegenüber der rohrförmigen Wandung ausgelenkt, so dass nicht nur die Fluidströmung durch die Zelle hindurch freigegeben ist, sondern die freigegebene Fluidströmung auch in Bezug auf die Strömungsrichtung beeinflussbar ist.

Ein weiterer Vorteil der erfindungsgemäßen medizinischen Vorrichtung besteht darin, dass durch die flexible Membran, die die wenigstens eine Klappe bildet, ermöglicht wird, dass mit der Fluidströmung transportierte Partikel in eine Vorzugsrichtung geleitet werden können. Beispielsweise kann durch die flexible Klappe im Bereich von Gefäßverzweigungen erreicht werden, dass in ein seitlich abzweigendes Gefäß eine Fluidströmung ermöglicht ist. Gleichzeitig kann die flexible Klappe verhindern, dass die Partikel, beispielsweise Thrombusbestandteile, in das seitliche Gefäß strömen. Auf diese Weise können die Partikel in Körperareale geführt werden, in denen ein Gefäßverschluss zu vergleichsweise geringen gesundheitlichen Beeinträchtigungen führt.

Gemäß einem nebengeordneten Aspekt beruht die Erfindung auf dem Gedanken, eine medizinische Vorrichtung, insbesondere einen Stent, mit einer rohrförmigen Wandung anzugeben, die einen axialen Hohlkanal bildet und Stege aufweist, die Zellen begrenzen. Dabei sind wenigstens zwei flexible Membrane vorgesehen, die in Längsrichtung des axialen Hohlkanals voneinander beabstandet angeordnet sind. Die flexiblen Membranen bilden jeweils wenigstens eine Klappe. Die Klappe weist ein erstes Ende auf, das mit wenigstens einem ersten Steg einer Zelle verbunden ist. Ferner weist die Klappe ein freies zweites Ende auf, das dem ersten Ende in Längsrichtung der Klappe gegenüber angeordnet ist. Die Klappe ist in eine Offenstellung und in eine Schließstellung überführbar. In der Offenstellung erstreckt sich die Klappe entlang der rohrförmigen Wandung. In der Schließstellung ist die Klappe relativ zur Wandung radial ausgelenkt und ragt in den axialen Hohlkanal hinein, um den axialen Hohlkanal ventilartig zumindest teilweise zu verschließen.

Im Unterschied zu der zuvor beschriebenen medizinischen Vorrichtung ist gemäß dem nebengeordneten Aspekt der Erfindung vorgesehen, den axialen Hohlkanal durch wenigstens zwei axial beabstandet voneinander angeordnete Klappen temporär zu verschließen. Dazu ist die Klappe an einem ersten Ende mit einem Steg der rohrförmigen Wandung fest verbunden. Das freie zweite Ende ist gegenüber dem ersten Ende beweglich, insbesondere in radialer Richtung bezüglich der rohrförmigen Wandung auslenkbar. In der Schließstellung verschließt die Klappe den axialen Hohlkanal. Die medizinische Vorrichtung gemäß dem nebengeordneten Aspekt der Erfindung hat den Vorteil, dass durch die wenigstens zwei Klappen, die einerseits axial voneinander beabstandet angeordnet sind und andererseits in der Schließstellung den axialen Hohlkanal zumindest teilweise verschließen, die Funktion von Venenklappen nachgebildet ist. Vorzugsweise sind die Klappen daher derart angepasst, dass die Klappen jeweils in Folge einer Fluidströmung durch den axialen Hohlkanal von der Schließstellung in die Offenstellung überführbar sind. Vorzugsweise sind die Klappen derart ausgerichtet bzw. angepasst, dass eine Fluidströmung in eine erste Axialrichtung die Klappe aus der Schließstellung in die Offenstellung überführt. Eine Fluidströmung in eine zweite, der ersten Axialrichtung entgegengesetzte Axialrichtung bewirkt vorteilhafterweise jeweils die Überführung der Klappe von der Offenstellung in die Schließstellung. Die Klappen ermöglichen also die Beeinflussung der Fluidströmung durch den axialen Hohlkanal derart, dass die Fluidströmung in eine einzige Richtung möglich ist. Eine Fluidströmung in entgegengesetzter Richtung wird blockiert. Alternativ kann die Überführung der Klappen von der Schließstellung in die Offenstellung auch manuell erfolgen.

Durch die zwei in axialer Richtung beabstandet angeordneten Membrane wird erreicht, dass die medizinische Vorrichtung als Venenklappenprothese einsetzbar ist, wobei wenigstens zwei Venenklappen gleichzeitig ersetzbar sind. Alternativ kann die medizinische Vorrichtung auch als vollwertiger Gefäßersatz bzw. als vollwertige Gefäßprothese eingesetzt werden. Beispielsweise können die Klappen auf einem Innenumfang der rohrförmigen Wandung angeordnet sein und der Außenumfang der rohrförmigen Wandung mit einer an sich bekannten Folie umschlossen sein, so dass eine schlauchartige Gefäßprothese mit einer Stützstruktur aus Stegen und einen axialen Hohlkanal verschließenden Klappen gebildet ist.

Die Klappe kann bei der Vorrichtung gemäß dem nebengeordneten Aspekt der Erfindung in der Offenstellung teilweise radial in den Hohlkanal hineinragen. Dadurch wird gewährleistet, dass die Klappe die Schließstellung selbsttätig einnimmt, wenn eine Fluidströmung entgegen der ersten Axialrichtung auf die Membran wirkt.

Die nachfolgenden bevorzugten Ausführungsformen einschließlich Wirkungen und Vorteilen beziehen sich, soweit sinnvoll, auf beide, unabhängig voneinander beanspruchte medizinische Vorrichtungen.

In einer ersten bevorzugten Ausführungsform weist die Zelle wenigstens einen ersten Knotenpunkt auf, der den ersten Steg mit einem zweiten Steg verbindet. Der erste Steg und der zweite Steg schließen im Bereich des Knotenpunktes einen Winkel ein. Das erste Ende der Klappe ist in der Nähe, insbesondere im Bereich des ersten Knotenpunkts, mit dem ersten Steg und/oder dem zweiten Steg verbunden. Durch die Anordnung des ersten Endes der Klappe im Bereich bzw. in der Nachbarschaft eines ersten Knotenpunktes wird erreicht, dass eine ausreichende Abdeckung der Zelle in einem expandierten Zustand der rohrförmigen Wandung sichergestellt ist. Gleichzeitig wird eine überhöhte Dehnung der flexiblen Membran, insbesondere der Klappe, beim Übergang vom komprimierten Zustand der rohrförmigen Wandung in den expandierten Zustand vermieden.

Das erste Ende der Klappe kann auf einem Außenumfang oder einem Innenumfang der rohrförmigen Wandung angeordnet sein. Insgesamt kann die Membran auf dem Außenumfang oder dem Innenumfang der rohrförmigen Wandung angeordnet sein. Die Anordnung der Membran bzw. des ersten Endes der Klappe auf dem Außenumfang der rohrförmigen Wandung hat den Vorteil, dass auf dem Außenumfang eine relativ gleichmäßige, glatte und flächige Außenhaut gebildet ist. Eine derartige Konfiguration ist beispielsweise bei der Behandlung von Stenosen, insbesondere bei vulnerablen Plaques, vorteilhaft, da die glatte Außenhaut eine Verletzung der vulnerablen Plaque vermeidet. Vorteilhaft bei der Anordnung der flexiblen Membran bzw. des ersten Endes der Klappe auf dem Innenumfang der rohrförmigen Wandung ist die verbesserte Anhaftung bzw. Fixierung der medizinischen Vorrichtung in einem Körperhohlgefäß. Bei dieser Konfiguration bilden die auf dem Außenumfang der rohrförmigen Wandung ausgebildeten Stege eine Gitterstruktur, die sich in, vorzugsweise gesunde, Gefäßwände leicht hineindrückt und die medizinische Vorrichtung somit fixiert. Auf dem Innenumfang der rohrförmigen Wandung bildet die flexible Membran zumindest in der Schließstellung der Klappen eine gleichmäßige Innenhaut, so dass ein strömungsoptimierter Hohlkanal ausgebildet ist. Durch die vergleichsweise glatte und gleichmäßige Innenhaut wird eine Thrombogenisierung im Bereich der medizinischen Vorrichtung und somit eine Restenose vermieden.

Bei einer weiteren bevorzugten Ausführungsform kann die Klappe eine im Wesentlichen blattartige Form aufweisen. Eine derartige Form ermöglicht eine zuverlässige Abdeckung der Zellen oder des axialen Hohlkanals. Die blattartige Form ermöglicht eine besonders vorteilhafte Faltung der Klappe bei der Überführung der rohrförmigen Wandung bzw. allgemein der medizinischen Vorrichtung vom expandierten Zustand in den komprimierten Zustand. Durch die blattförmige Form der Klappe wird daher erreicht, dass die rohrförmige Wandung auf einem relativ kleinen Querschnittsdurchmesser komprimierbar ist.

Vorzugsweise weist die Klappe eine Strukturierung, insbesondere eine Porenstruktur oder eine Rillenstruktur oder eine Fleecestruktur, auf. Die Strukturierung der Klappe ermöglicht eine Endothelialisierung. Das bedeutet, dass sich durch die Strukturierung an der Klappe Endothelzellen anlagern können. Eine schnelle Anlagerung von Endothelzellen ist erwünscht, da auf diese Weise das Thrombose- bzw. Restenoserisiko reduziert wird. Durch die Strukturierung kann auch erreicht werden, dass kleinere Nebengefäße mit einer Fluidströmung beaufschlagt werden, wobei die Fluidströmung durch die Strukturierung der Klappe hindurch möglich ist. Dies ist insbesondere vorteilhaft, wenn der Querschnittsdurchmesser des Nebengefäßes kleiner als die Längs- oder Umfangserstreckung der Klappe ist. Überdies erhöht die Strukturierung die Flexibilität der Klappe bzw. wirkt Materialspannungen in der Klappe, die beispielsweise durch eine Dehnung der Klappe ausgelöst werden, entgegen.

Gemäß einer bevorzugten Ausgestaltung weist die Klappe eine Faltlinie auf, die sich zumindest abschnittsweise von dem ersten Ende zum freien zweiten Ende erstreckt. Die Faltlinie gibt eine bevorzugte Faltung vor. Die Faltlinie verläuft vorzugsweise in Längsrichtung der Klappe. Besonders vorteilhaft ist es, wenn die Faltlinie mit zwei in Längsrichtung der rohrförmigen Wandung benachbart angeordneten Knotenpunkten fluchtet. Dadurch wird erreicht, dass sich die Klappe bevorzugt radial durch die Zelle hindurchfaltet, wenn die medizinische Vorrichtung vom expandierten Zustand in den komprimierten Zustand überführt wird. Dadurch ist eine besonders kleine komprimierte Form der medizinischen Vorrichtung möglich. Die Faltlinie kann die Klappe in einen ersten Klappenflügel und in einen zweiten Klappenflügel unterteilen. Vorzugsweise sind der erste Klappenflügel mit dem ersten Steg der Zelle und der zweite Klappenflügel mit dem zweiten Steg der Zelle verbunden. Auf diese Weise wird sichergestellt, dass sich die Klappe gleichmäßig entlang der Faltlinie faltet.

Die Faltlinie kann eine Nut und/oder einen Spalt umfassen, so dass das Falten der Klappen beim Übergang der medizinischen Vorrichtung vom expandierten Zustand in den komprimierten Zustand erleichtert ist. Gemäß einer bevorzugten Ausgestaltung kann der Spalt den ersten Klappenflügel zumindest abschnittsweise von dem zweiten Klappenflügel trennen. Der erste Klappenflügel kann mit dem zweiten Klappenflügel verbunden bzw. einteilig ausgebildet sein, wobei zwischen dem ersten Klappenflügel und dem zweiten Klappenflügel ein Abschnitt vorgesehen ist, der den Spalt umfasst. Im Bereich des Spaltes sind der erste Klappenflügel und der zweite Klappenflügel voneinander beabstandet angeordnet. Vorzugsweise ist der Spalt in Längsrichtung der Klappe angeordnet. Der Spalt kann in Richtung des ersten Knotenpunktes, in dessen Nähe der erste und zweite Klappenflügel mit jeweils einem ersten bzw. zweiten Steg verbunden ist, offen sein. Der Spalt kann den ersten Klappenflügel vollständig von dem zweiten Klappenflügel trennen, so dass der erste Klappenflügel von dem zweiten Klappenflügel vollständig beabstandet angeordnet ist. In diesem Fall bildet die Klappe zwei einzelne Klappenteile, nämlich den ersten Klappenflügel und den zweiten Klappenflügel. Auf diese Weise wird die Faltung bzw. die Anordnung der Klappe im komprimierten Zustand der medizinischen Vorrichtung weiter verbessert. Ferner wird durch die Aufteilung der Klappe in zwei separate Klappenflügel die Überführung von der Schließstellung in die Offenstellung erleichtert. Insgesamt kann somit die Ventilfunktion verbessert werden.

Wie zuvor vorteilhaft erläutert, kann die Klappe selbsttätig von der Schließstellung in die Offenstellung überführbar sein.

Das freie Ende der Klappe kann in einer bevorzugten Ausgestaltung in der Schließstellung in die Zelle hineinragen, insbesondere innerhalb der Zelle angeordnet sein, oder die Zelle überlappen. Dabei erfolgt die Überlappung der Zelle zumindest in Längsrichtung der medizinischen Vorrichtung. Das bedeutet, dass in Umfangsrichtung der rohrförmigen Wandung Teilbereiche der Zelle durch die Klappe nicht bedeckt sein können. Wenn die Klappe die Zelle überlappt, erstreckt sich das freie Ende der Klappe in Längsrichtung bzw. Axialrichtung der rohrförmigen Wandung über die Zelle hinaus. Durch die Überlappung wird sichergestellt, dass die Zelle im expandierten Zustand der rohrförmigen Wandung bzw. allgemein der medizinischen Vorrichtung zuverlässig abgedeckt ist, wenn die Klappe die Schließstellung eingenommen hat. Alternativ kann in der Schließstellung der Klappe die Zelle im expandierten Zustand der rohrförmigen Wandung bzw. der medizinischen Vorrichtung teilweise unbedeckt sein.

Es ist möglich, dass sich die Klappe in der Schließstellung in Längsrichtung oder in Umfangsrichtung der rohrförmigen Wandung erstreckt. Die Erstreckung der Klappe in der Schließstellung in Längsrichtung bzw. Axialrichtung der rohrförmigen Wandung ist besonders bevorzugt.

Bei einer bevorzugten Ausführungsform der medizinischen Vorrichtung weist die Membran mehrere Klappen auf. Die mehreren Klappen erstrecken sich in der Schließstellung entlang der rohrförmigen Wandung. Jeweils ein freies Ende einer ersten Klappe überlappt wenigstens ein, insbesondere zwei, erste Enden einer benachbarten Klappe dachziegelartig. Die dachziegelartige Überlappung der Klappen ist zumindest in einem komprimierten Zustand der rohrförmigen Wandung bzw. allgemein der medizinischen Vorrichtung erkennbar. Vorzugsweise ist die dachziegelartige Überlappung auch im expandierten Zustand der rohrförmigen Wandung eingestellt. Die dachziegelartige Überlappung kann sich zumindest in Umfangsrichtung im komprimierten Zustand der rohrförmigen Wandung einstellen. Bei der dachziegelartigen Überlappung überlappt wenigstens eine erste Klappe wenigstens eine zweite Klappe, vorzugsweise zwei zweite Klappen. Die erste Klappe kann sich dabei in Längsrichtung der rohrförmigen Wandung erstrecken, wobei das zweite, freie Ende im Wesentlichen zwischen zwei zweiten, in Längsrichtung der rohrförmigen Wandung benachbart angeordneten Klappen erstreckt.

Mit anderen Worten umfasst die medizinische Vorrichtung nach diesem Ausführungsbeispiel mehrere in axialer Richtung der rohrförmigen Wandung benachbart angeordnete Reihen von Klappen, wobei die Klappen jeweils unmittelbar in axialer Richtung aufeinanderfolgender Reihen zueinander versetzt angeordnet sind. Somit überlappen die ersten Klappen einer ersten Reihe jeweils zwei zweite Klappen einer zweiten Reihe. Insbesondere überlappen die zweiten, freien Enden der ersten Klappen einer ersten Reihe im komprimierten Zustand der rohrförmigen Wandung jeweils zwei erste, fixierte Enden zweier zweiter Klappen einer zweiten Reihe. Es ist auch möglich, dass die zweiten, freien Enden der ersten Klappen einer ersten Reihe zwei zweite, freie Enden zweier zweiter Klappen einer zweiten Reihe überlappen. Mit anderen Worten können die Klappen benachbarter Reihen gegenläufig angeordnet sein. Dasselbe gilt analog für eine Anordnung der Klappen in Umfangsrichtung der rohrförmigen Wandung. Die erste Reihe und die zweite Reihe von Klappen erstrecken sich in diesem Fall in Längsrichtung. Die ersten Klappen der ersten Reihe überlappen dabei wenigstens eine, insbesondere zwei zweite Klappen einer in Umfangsrichtung benachbart angeordneten zweiten Reihe von Klappen. Im expandierten Zustand der rohrförmigen Wandung können die ersten Klappen der ersten Reihe wenigstens die Zellen der zweiten Reihe teilweise überlappen. Dabei können sich die ersten Klappen der ersten Reihe zwischen zwei zweite Klappen der zweiten Reihe erstrecken.

Gemäß einer bevorzugten Ausführungsform der medizinischen Vorrichtung nach dem nebengeordneten Aspekt der Erfindung bilden die wenigstens zwei Membranen jeweils eine Mehrfachklappe. Die Mehrfachklappe ist von einer Schließstellung in eine Offenstellung überführbar. Vorzugsweise weist die Mehrfachklappe wenigstens zwei Klappen auf, die bezogen auf eine Längsachse des axialen Hohlraums radial gegenüber angeordnet sind. Die wenigstens zwei Klappen erstrecken sich in der Schließstellung in den axialen Hohlraum, um den axialen Hohlraum ventilartig zu verschließen. Im Hinblick auf die Fluiddynamik ist die Mehrfachklappe besonders vorteilhaft.

Die freien, zweiten Enden der Klappen der Mehrfachklappe können sich in der Schließstellung berühren. Dadurch ist gewährleistet, dass der axiale Hohlraum sicher verschlossen ist. Vorzugsweise ist der axiale Hohlraum in der Schließstellung der Mehrfachklappe bzw. allgemein der Klappe fluiddicht verschlossen.

Vorzugsweise weist eine Zelle zwei Klappen auf, die in einem expandierten Zustand der Zelle in Gegenüberstellung und in einem komprimierten Zustand der Zelle seitlich nebeneinander angeordnet sind. Bezogen auf die Einzelzelle hat diese Anordnung den Vorteil, dass ein großer Bereich der Zelle abgedeckt ist, ohne dass die Zelle zu lang ist. Bezogen auf eine Zelle mit mehr als zwei Klappen, insbesondere mit 4 Klappen besteht der Vorteil darin, dass das Crimpen vereinfacht wird, da die Abdeckung bzw. die Klappen mit der Struktur und nicht untereinander beim Crimpen überlappen.

Bei einer weiteren Ausführungsform ist zumindest im Bereich des ersten Endes der Klappe eine Porenstruktur, insbesondere eine Perforierung der Klappe ausgebildet, die sich bis in den Bereich des ersten Steges erstreckt, wobei das erste Ende abschnittsweise mit dem ersten Steg verbunden ist derart, dass die Porenstruktur des ersten Endes im losen Bereich oder in den losen Bereichen verformbar ist. Damit wird eine Verformung der Abdeckung bzw. der Klappe vermieden oder zumindest verringert.

Es wird darauf hingewiesen, dass die zuvor beschriebenen vorteilhaften Ausgestaltungen der Erfindung generell den expandierten Zustand der medizinischen Vorrichtung beschreiben, wenn nicht anderweitig explizit erläutert. Dies gilt auch für die nachfolgende Beschreibung bevorzugter Ausführungsbeispiele.

Die Erfindung wird im Folgenden anhand von Ausführungsbeispielen unter Bezugnahme auf die beigefügten, schematischen Zeichnungen näher erläutert. Darin zeigen:
- Fig. 1 und 2: eine Draufsicht auf eine rohrförmige Wandung einer erfindungsgemäßen medizinischen Vorrichtung nach jeweils einem bevorzugten Ausführungsbeispiel;
- Fig. 3 bis 9: einen Längsschnitt durch eine erfindungsgemäße medizinische Vorrichtung nach jeweils einem bevorzugten Ausführungsbeispiel in unterschiedlichen Einbaulagen;
- Fig. 10 bis 14: eine Detailansicht einer Klappe einer erfindungsgemäßen medizinischen Vorrichtung nach jeweils einem bevorzugten Ausführungsbeispiel;
- Fig. 15: eine Draufsicht auf eine rohrförmige Wandung einer erfindungsgemäßen medizinischen Vorrichtung nach einem weiteren bevorzugten Ausführungsbeispiel;
- Fig. 16a und 16b: jeweils einen Querschnitt durch eine medizinische Vorrichtung nach dem nebengeordneten Aspekt der Erfindung gemäß einem bevorzugten Ausführungsbeispiel in der Einbaulage mit unterschiedlichen Stellungen der Klappen;
- Fig. 17a bis 17c: jeweils eine Querschnittsansicht durch eine rohrförmige Wandung mit einer Klappe nach einem bevorzugten Ausführungsbeispiel;
- Fig. 18: eine Detailansicht einer Klappe einer erfindungsgemäßen medizinischen Vorrichtung nach einem bevorzugten Ausführungsbeispiel;
- Fig. 19: eine Detailansicht einer Klappe einer erfindungsgemäßen medizinischen Vorrichtung nach einem weiteren bevorzugten Ausführungsbeispiel;
- Fig. 20a: eine Detailansicht einer Mehrfachklappe einer erfindungsgemäßen medizinischen Vorrichtung nach einem weiteren bevorzugten Ausführungsbeispiel im expandierten Zustand;
- Fig. 20b: das Ausführungsbeispiel gemäß Fig. 20a im komprimierten Zustand;
- Fig. 20c: einen Ausschnitt aus einer Gitterstruktur mit Zellen gemäß dem Ausführungsbeispiel gemäß Fig. 20a; und
- Fig. 21: eine Detailansicht einer Klappe einer erfindungsgemäßen medizinischen Vorrichtung nach einem weiteren bevorzugten Ausführungsbeispiel mit einer bis zum Steg sich erstreckenden Perforierung.

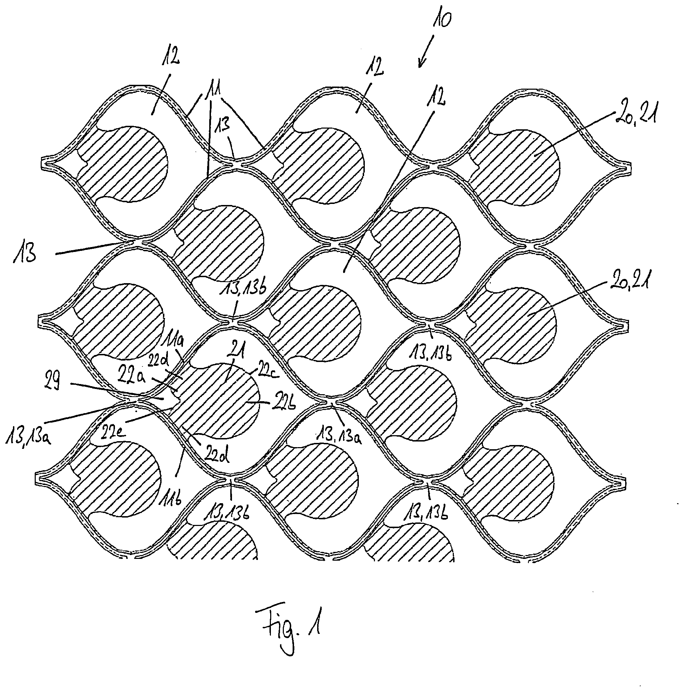

Fig. 1 zeigt den expandierten Zustand einer rohrförmigen Wandung 10 bzw. allgemein der medizinischen Vorrichtung. Dasselbe gilt für alle Ausführungsbeispiele, die in den Fig. 1 bis 17c dargestellt sind. Die rohrförmige Wandung 10 bzw. der Ausschnitt der rohrförmigen Wandung 10 ist aus Gründen der Übersichtlichkeit in Fig. 1 im aufgeklappten bzw. flächig ausgebreiteten Zustand dargestellt. Die axiale Längsachse der rohrförmigen Wandung 10 verläuft in den Fig. 1, 2, 3, 7 und 10 bis 15 im Wesentlichen horizontal in der Zeichenebene.

Der Detailausschnitt der Wandung 10 in Fig. 1 zeigt mehrere Stege 11, die jeweils zwei Knotenpunkte 13 miteinander verbinden. Im expandierten Zustand sind die Stege 11 S-förmig ausgebildet. Jeweils vier Stege 11 sind in einem Knotenpunkt 13 miteinander verbunden. Jeweils vier Stege 11 begrenzen eine Zelle 12 der rohrförmigen Wandung 10. Die Zellen 12 sind vollständig von Stegen 11 begrenzt. Die rohrförmige Wandung 10 gemäß Fig. 1 bildet daher eine Closed-Cell-Struktur. Alternativ ist es möglich, dass die rohrförmige Wandung 10 eine Open-Cell-Struktur aufweist, wobei wenigstens zwei Stege 11 in einem Knotenpunkt 13 frei beweglich, also nicht miteinander verbunden, sind. Die Stege 11 können in diesem Fall vielmehr Endbögen bilden, die bei der Überführung der rohrförmigen Wandung bzw. allgemein einer Gitterstruktur, die durch die Stege 11 gebildet ist, vom komprimierten Zustand in den expandierten Zustand ihre Position bezüglich der Gitterstruktur ändern können. Für die im Rahmen dieser Anmeldung beschriebenen Ausführungsbeispiele ist die Closed-Cell-Struktur bevorzugt.

Insgesamt bilden die Stege 11 die Gitterstruktur bzw. die rohrförmige Wandung 10. Mit anderen Worten weist die medizinische Vorrichtung eine Trägerstruktur bzw. Stützstruktur auf, die durch die in den Knotenpunkten 13 verbundenen Stege 11 gebildet ist.

Die Zellen 12 umfassen jeweils einen ersten Steg 11a und einen zweiten Steg 11b, wobei der erste und zweite Steg 11a, 11b in einem ersten Knotenpunkt 13a miteinander verbunden sind. Der erste Steg 11a und der zweite Steg 11b erstrecken sich vom ersten Knotenpunkt 13a zu jeweils einem zweiten Knotenpunkt 13b, wobei die Knotenpunkte 13a und 13b in Längsrichtung der rohrförmigen Wandung 10 benachbart und in Umfangsrichtung versetzt angeordnet sind. Dies gilt insbesondere im expandierten Zustand der rohrförmigen Wandung 10. Ausgehend vom ersten Knotenpunkt 13a schließen der erste Steg 11a und der zweite Steg 11b einen Winkel ein. Die ersten Knotenpunkte 13a benachbarter Zellen 12 fluchten zueinander. Dies gilt sowohl für die Längsrichtung der rohrförmigen Wandung 10, als auch für die Umfangsrichtung. Dasselbe gilt für die zweiten Knotenpunkte 13b jeweils benachbarter Zellen 12.

Die rohrförmige Wandung 10 weist ferner eine flexible Membran 20 auf. Die flexible Membran bildet wenigstens eine Klappe 21. Gemäß dem Ausführungsbeispiel nach Fig. 1 sind mehrere Klappen 21 vorgesehen, wobei jeweils eine Klappe 21 einer einzigen Zelle 12 zugeordnet ist. Die Klappe 21 umfasst ein erstes Ende 22a, das mit dem ersten Steg 11a und dem zweiten Steg 11b verbunden ist. Das erste Ende 22a ist vorzugsweise stoffschlüssig mit dem ersten Steg 11a verbunden. Die Verbindung mit dem zweiten Steg 11b erfolgt vorzugsweise ebenfalls stoffschlüssig. Das erste Ende 22a der Klappe 21 ist in der Nähe eines Knotenpunkts 13, insbesondere des Knotenpunkts 13a, angeordnet.

In diesem Zusammenhang wird darauf hingewiesen, dass alle Punkte eines Stegs 11, die von dem Knotenpunkt 13 eine Entfernung aufweisen, die höchstens der halben Länge des Stegs 11 entspricht, als Punkte bzw. Bereiche angesehen werden, die sich in der Nähe des Knotenpunkts 13 befinden. Dabei bezieht sich die Entfernung auf die Wegstrecke zwischen dem zu bestimmenden Punkt und dem Knotenpunkt 13 entlang des Stegs 11. Mit anderen Worten gehört die Hälfte des Stegs 11, die dem Knotenpunkt 13 zugewandt bzw. im Knotenpunkt 13 mit einem weiteren Steg 11 verbunden ist, zur Nähe dieses Knotenpunkts 13.

Die Klappe 21 weist ferner ein freies, zweites Ende 22b auf, das in Längsrichtung der Klappe 21 dem ersten Ende 22a gegenüber angeordnet ist. Die Klappe 21 erstreckt sich bei dem Ausführungsbeispiel gemäß Fig. 1 in Längsrichtung der rohrförmigen Wandung 10. In Längsrichtung der Klappe 21 bzw. der rohrförmigen Wandung 10 sind das erste Ende 22a und das zweite Ende 22b der Klappe 21 einander nachgeordnet. Insgesamt fluchtet die Klappe 21 mit den in Längsrichtung der Wandung 10 einander nachgeordneten ersten Knotenpunkten 13a benachbarter Zellen 12.

Wie in Fig. 1 weiter dargestellt weist die Klappe 21 im Wesentlichen eine blattartige Form auf. Das zweite freie Ende 22b bildet insbesondere eine gekrümmte freie Kante 22c der Klappe 21. Die freie Kante 22c erstreckt sich über das freie, zweite Ende 22b hinaus und ist durch den ersten und zweiten Steg 11a, 11b begrenzt. Die Form der freien Kante 22c zwischen dem ersten Steg 11a und dem zweiten Steg 11b entspricht im Wesentlichen einem Omega. Das erste Ende 22a umfasst zwei Halteabschnitte 22d, die jeweils mit dem ersten Steg 11a oder dem zweiten Steg 11b verbunden sind. Zwischen den Halteabschnitten 22d ist im Bereich des ersten Endes 22a eine innere Kante 22e ausgebildet. Die innere Kante 22e erstreckt sich entlang der Halteabschnitte 22d von dem ersten Steg 11a zum zweiten Steg 11b. Dabei weist die innere Kante 22e in den beiden Halteabschnitten 22d jeweils eine gegenläufige Krümmung auf. Die innere Kante 22e bildet somit einen zeltdachförmigen Einschnitt in die Klappe 21. Zusammen mit dem ersten Steg 11a und dem zweiten Steg 11b schließt die innere Kante 22e eine Faltöffnung 29 ein. Die Faltöffnung 29 weist im Wesentlichen die Form eines Drachenvierecks bzw. Deltoids mit nach innen gekrümmten Seitenkanten auf. Dabei erstreckt sich die längere Diagonale des Drachenvierecks in Längsrichtung der Klappe 21 bzw. der rohrförmigen Wandung 10.

Bei dem Ausführungsbeispiel gemäß Fig. 1 erstreckt sich die Klappe 21 in der Schließstellung in die Zelle 12. Das bedeutet, dass die Klappe 21 die Zelle 12 in der Schließstellung teilweise überdeckt. In Fig. 1 ist gut zu erkennen, dass die Klappe 21 einen mittleren Bereich der Zelle 12 bedeckt. Ein äußerer Bereich der Zelle 12, der zwischen den Stegen 11 und der freien Kante 22c ausgebildet ist, ist fluiddurchlässig bzw. nicht bedeckt. Es ist auch möglich, dass die Klappe 21 derart geformt ist, dass die Zelle 12 in der Schließstellung der Klappe 21 und im expandierten Zustand der rohrförmigen Wandung 10 vollständig überdeckt ist. Beispielsweise kann auch die Faltöffnung 29 durch die Membran 20 bzw. die Klappe 21 bedeckt sein.

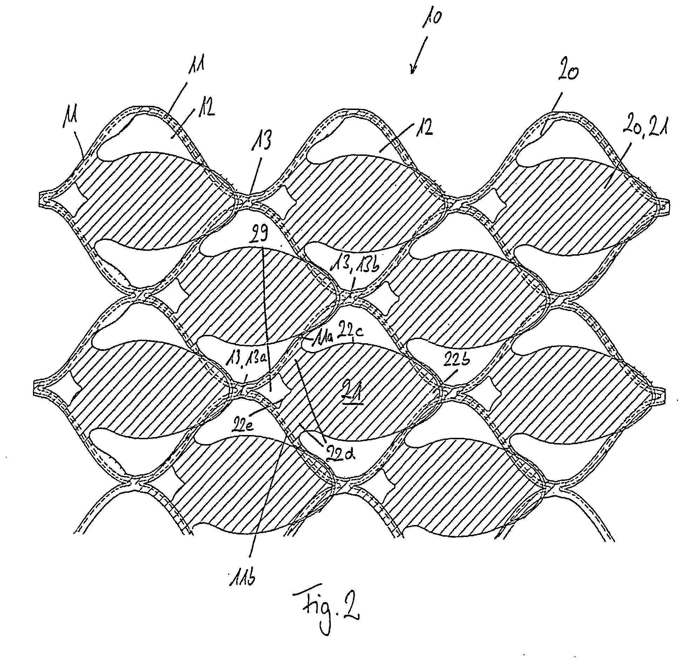

Ein weiteres Ausführungsbeispiel der medizinischen Vorrichtung, bei dem die Klappe 21 einen größeren Bereich der Zelle 12 überdeckt, ist in Fig. 2 dargestellt. Das Ausführungsbeispiel gemäß Fig. 2 entspricht im Wesentlichen dem Ausführungsbeispiel gemäß Fig. 1, wobei die Klappe 21 eine vergleichsweise größere Längserstreckung aufweist und die Zelle 12 überlappt. Das bedeutet, dass das zweite, freie Ende 22b der Klappe 21 in der Schließstellung im Bereich des Knotenpunkts 13, insbesondere des ersten Knotenpunkts 13a einer benachbarten Zelle 12, auf den Stegen 11 aufliegend angeordnet ist. Die Klappe 21 überlappt also die Zelle 12. Zusätzlich ist bei dem Ausführungsbeispiel gemäß Fig. 2 vorgesehen, die Membran 20 auf Bereiche des ersten Stegs 11a und des zweiten Stegs 11b auszudehnen, die entfernt vom ersten Knotenpunkt 13a angeordnet sind. Dabei bildet die Membran 20 entlang des Stegs 11 eine flügelartige Verbreiterung. Die flügelartige Verbreiterung an sich ist im deutschen Patent Nr. 10 2008 010 507 ausführlich beschrieben, das auf die Anmelderin zurückgeht. Eine Kombination der medizinischen Vorrichtung mit den darin beschriebenen flügelartigen Verbreiterungen der Stege 11 wird hiermit explizit offenbart.

Die Membran 20 kann auf dem Außenumfang 14a der rohrförmigen Wandung 10 angeordnet sein. Die Klappen 21 erstrecken sich dabei über den Außenumfang 14a. Alternativ kann die Membran 20 auf einem Innenumfang 14d der rohrförmigen Wandung 10 angeordnet sein. In diesem Fall erstrecken sich die Klappen 21 entlang des Innenumfangs 14b. In beiden Fällen können sich die Klappen 21 in Längsrichtung der Wandung 10 erstrecken. Es ist auch möglich, dass sich die Klappen 21 in Umfangsrichtung der Wandung 10 erstrecken. Ferner können sich die Klappen 21 jeweils in dieselbe Richtung erstrecken. Das bedeutet, dass in der Schließstellung jeweils ein freies, zweites Ende 22b einer ersten Klappe 21a mit einem fixierten ersten Ende 22a einer zweiten Klappe 21b fluchtet bzw. dieses überlappt. Alternativ können die Klappen 21 benachbarter Zellen 12 gegenläufig angeordnet sein. Beispielsweise können eine erste Klappe 21a und eine in Längsrichtung der ersten Klappe 21a benachbarte zweite Klappe 21b jeweils ein freies zweites Ende 22b aufweisen, wobei die freien zweiten Enden 22b zueinander benachbart oder sich überlappend angeordnet sind.

Generell sind die Klappen 21 bzw. die Membran 20 flexibel. Insbesondere sind die Klappen 21 derart flexibel, dass das zweite, freie Ende 22b der Klappe 21 gegenüber dem ersten, fixierten Ende 22a radial auslenkbar ist. Die Klappe 21a ist also derart flexibel, dass die Klappe 21a in einer Offenstellung überführbar ist, in der Klappe 21 die Zelle 12 nicht bedeckt. Die Zelle 12 ist in der Offenstellung der Klappe 21 im Wesentlichen frei durchgängig. Die Klappe 21, insbesondere das zweite freie Ende 22b, ist in der Offenstellung ausgelenkt bzw. in radialer Richtung bezüglich der rohrförmigen Wandung 10 gebogen. Dabei kann die Klappe 21 sowohl nach außen, als auch nach innen gekrümmt sein. In der Offenstellung weist das freie Ende 22b also entweder nach radial außen oder nach radial innen bezüglich der rohrförmigen Wandung 10. Dabei ist es unerheblich, ob die Klappe 21 mit dem ersten Ende 22a auf dem Außenumfang 14a oder auf dem Innenumfang 14b der rohrförmigen Wandung angeordnet ist. Bevorzugt ist die Klappe 21 derart angepasst, dass eine Auslenkung nach radial außen erfolgt, wenn das erste Ende 22a der Klappe 21 mit dem Außenumfang 14a der rohrförmigen Wandung 10 verbunden ist. Analog ist die Klappe 21 vorzugsweise derart angepasst, dass eine Auslenkung nach radial innen bezüglich der rohrförmigen Wandung 10 erfolgt, wenn die Klappe 21 mit dem ersten Ende 22a auf den Innenumfang der rohrförmigen Wandung 10 angeordnet ist.

Im Allgemeinen umfasst die medizinische Vorrichtung zwei Hauptfunktionselemente. Einerseits ist eine tragende Struktur, insbesondere in Form der rohrförmigen Wandung 10, die vorzugsweise eine Gitterstruktur aus Stegen 11 umfasst, vorgesehen, die die Stabilität bzw. Kraft in radialer Richtung sicherstellt. Die tragende Struktur kann im Wesentlichen eine stentartige Geometrie aufweisen. Andererseits weist die medizinische Vorrichtung eine Abdeckung, insbesondere in Form der Membran 20, auf, die Klappen 21 bildet. Die Klappen 21 ragen in die Zellen 12 hinein bzw. überlappen diese. In der Schließstellung wird die Zelle 12 durch die Klappe 21 wenigstens teilweise, insbesondere vollständig, abgedeckt. Die flexible Klappe 21 weist eine Ventilfunktion auf. Dazu weist die Klappe das erste fixierte Ende 22a und das zweite freie bzw. lose Ende 22b auf.

Nachfolgend werden besonders vorteilhafte konstruktive Merkmale der Membran 20 erläutert:
Die Funktion der Membran 20 bzw. der Klappe 21 besteht insbesondere darin, die Zelle 12 nach Art eines Ventils freizugeben. Die Freigabe kann insbesondere durch einen Druckgradienten bewirkt sein, der sich zwischen den Seiten der Zelle, insbesondere einem inneren axialen Hohlkanal 30 der rohrförmigen Wandung 10 bzw. der medizinischen Vorrichtung und einem Bereich radial außerhalb der rohrförmigen Wandung 10 einstellt. Im Allgemeinen ist es vorteilhaft, wenn die flexible Klappe 21 dadurch in die Offenstellung bzw. Schließstellung überführbar ist, dass eine Fluidströmung auf die Klappe 21 wirkt. Vorteilhaft ist eine hohe Flexibilität der Klappe 21. Die Klappe 21 kann im Wesentlichen als Folie ausgebildet sein. Die Wandstärke der Klappe 21 kann vergleichsweise klein sein. Die vergleichsweise kleine Wandstärke ermöglicht nicht nur eine verbesserte bzw. erleichterte Ventilfunktion, sondern auch eine Verbesserung beim Crimpen der rohrförmigen Wandung 10, da sich die relativ dünne Klappe 21 in die Zelle 12 hineinfaltet und durch die dünne Wandstärke gewährleistet ist, dass ein relativ kleiner gecrimpter Querschnittsdurchmesser für die rohrförmige Wandung 10 erreichbar ist. Um dies zu erreichen, ist vorteilhaft vorgesehen, dass die Membran 20, insbesondere die Klappe 21, eine Wandstärke aufweist, die höchstens 15 µm, insbesondere höchstens 10 µm, insbesondere höchstens 8 µm, insbesondere höchstens 6 µm, insbesondere höchstens 4 µm, insbesondere höchstens 2 µm, insbesondere höchstens 3 µm, insbesondere höchstens 2 µm, beträgt.

Durch die relativ kleine Wandstärke der Klappe 21 wird ferner ermöglicht, dass die Klappe 21 bei der Durchströmung der Zelle 12 mit einem Körperfluid, insbesondere Blut, flattert. Dabei können sich vorteilhaft turbulente Strömungsverhältnisse im Bereich der Klappe 21 bilden, so dass beispielsweise ein Blutstau vermieden bzw. einer Blutgerinnung, insbesondere Thrombogenisierung, entgegengewirkt wird. Durch das Flattern der Klappe 21 wird vielmehr ein konstanter, kontinuierlicher Durchfluss in der Offenstellung erreicht.

Vorzugsweise erstreckt sich bzw. überdeckt die Klappe 21 die gesamte Zelle 12. Zumindest überdeckt die Klappe 21 wenigstens 10 %, insbesondere wenigstens 20 %, insbesondere wenigstens 30 %, insbesondere wenigstens 40 %, insbesondere wenigstens 50 %, insbesondere wenigstens 60 %, insbesondere wenigstens 70 %, insbesondere wenigstens 80 %, der Zelle 12 im expandierten Zustand. Der größtmögliche Kreisdurchmesser, der in einem von der Klappe 21 freigegebenen Bereich erkennbar ist, beträgt höchstens 80 %, insbesondere höchstens 60 %, insbesondere höchstens 40 %, insbesondere höchstens 30 %, insbesondere höchstens 20 %, insbesondere höchstens 10 %, der Größe der Zelle 12, wobei die Größe der Zelle 12 dem Durchmesser des größtmöglichen Kreises entspricht, der in der Zelle 12 anordenbar ist.

Die Klappe 21 kann ferner eine Strukturierung, insbesondere Oberflächenstrukturierung aufweisen, die beispielsweise die Endothelialisierung der medizinischen Vorrichtung fördert. Die Strukturierung kann beispielsweise eine Porenstruktur 28 umfassen. Die Strukturierung kann mehrere Öffnungen aufweisen, die in die Klappe 21 eingebracht sind und die Klappe 21 in radialer Richtung bezogen auf die Gitterstruktur 10 vollständig durchdringen.

Im Allgemeinen ändert sich der Abstand zwischen zwei in Längsrichtung der medizinischen Vorrichtung benachbart angeordneten Knotenpunkten, wenn sich der Zustand der Wandung 10 ändert, also die Wandung 10 vom radial komprimierten in den radial expandierten Zustand und umgekehrt übergeht. Insbesondere der Übergang vom radial expandierten in den radial komprimierten Zustand der Wandung 10 bewirkt eine Verlängerung des Abstandes zwischen in Längsrichtung der medizinischen Vorrichtung benachbart angeordneten Knotenpunkten 13, 13a. Dabei können sich die S-förmigen Stege 11 verformen, was zu einer Dehnung der Klappe 21 führen kann. Durch die Strukturierung, insbesondere die musterartige Porenstruktur 28, wird die Dehnung bzw. das Aufspannen der Klappe 21 erleichtert. Insbesondere verhindert die Porenstruktur 28, dass es zu Spannungen in der Klappe 21 bzw. allgemein der Membran 20 kommt.

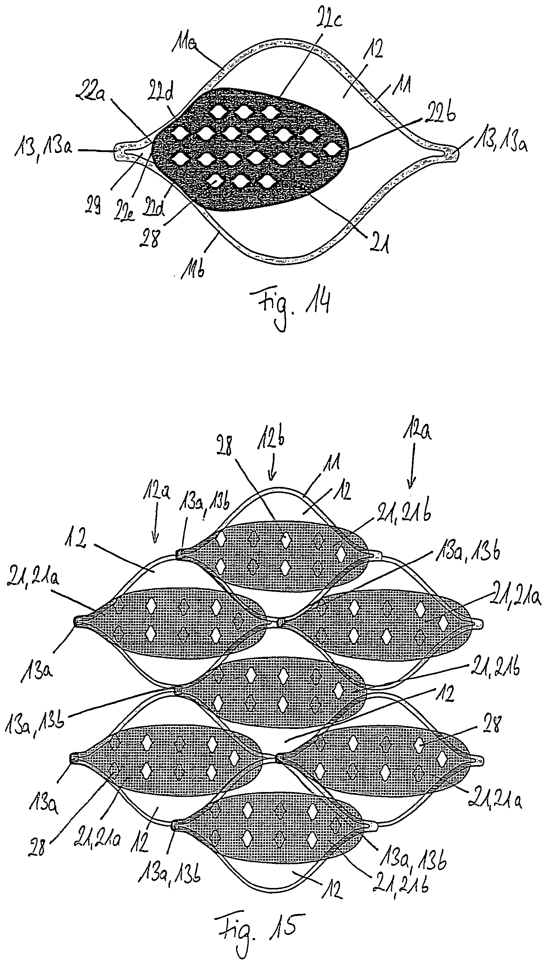

Vorzugsweise weist die Porenstruktur 28 Poren auf, die rautenförmig ausgebildet sind. Die rautenförmigen Poren umfassen vorteilhafterweise zwei gegenüberliegend angeordnete Ecken, die einen relativ großen Winkel aufweisen. Beispielsweise kann der Winkel wenigstens 90°, insbesondere wenigstens 120°, insbesondere wenigstens 130°, insbesondere wenigstens 140°, insbesondere wenigstens 150°, insbesondere wenigstens 160°, insbesondere wenigstens 170°, betragen. Die Ecken mit dem größeren Eckenwinkel sind vorzugsweise in Umfangsrichtung der rohrförmigen Wandung 10 gegenüberliegend angeordnet. Die Poren der Porenstruktur 28 sind vorzugsweise musterartig, also regelmäßig, über die Klappe 21 verteilt angeordnet, wie beispielsweise in den Fig. 14 und 15 dargestellt.

Die Poren der Porenstruktur 28 können auch schlitzförmig ausgebildet sein. Die in Umfangsrichtung der rohrförmigen Wandung 10 gegenüberliegend angeordneten Eckenwinkel der Poren betragen also beinahe 180° bzw. genau 180°. Die Spaltbreite der schlitzförmigen Poren kann höchstens 300 µm, insbesondere höchstens 200 µm, insbesondere höchstens 100 µm, insbesondere höchstens 50 µm, betragen. Im Allgemeinen bewirkt der verhältnismäßig große Winkel der gegenüberliegend angeordneten Ecken der Poren der Porenstruktur 28 eine besonders große Flexibilität der Klappe 21 bzw. allgemein der Membran 20. Eine Dehnung der Membran 20 bzw. der Klappe 21 wird somit effektiv vermieden oder zumindest reduziert. Die vorstehend beschriebene Porenstruktur 28 kann mit allen Ausführungsbeispielen kombiniert werden.

Die Klappe 21 ist im Allgemeinen derart flexibel, dass in der Offenstellung eine Auslenkung ermöglicht ist, die in Relation zur Schließstellung wenigstens 20°, insbesondere wenigstens 30°, insbesondere wenigstens 45°, insbesondere wenigstens 60°, insbesondere wenigstens 90° beträgt. Dabei erfolgt die Auslenkung im elastisch verformbaren Bereich der Klappe 21. Bei der Auslenkung der Klappe 21 in der Offenstellung ist die Klappe 21 gekrümmt. Der maximale Krümmungsradius bzw. der Radius der Krümmung an der am stärksten gekrümmten Stelle der Klappe 21 beträgt vorzugsweise höchstens 2 mm, insbesondere höchstens 1,5 mm, insbesondere höchstens 1 mm, insbesondere höchstens 0,8 mm, insbesondere höchstens 0,6 mm, insbesondere höchstens 0,4 mm, insbesondere höchstens 0,2, insbesondere höchstens 0,1 mm, insbesondere höchstens 0,05 mm. Generell ist die Klappe 21 vorzugsweise derart flexibel, dass sie in Abhängigkeit der physiologischen Gegebenheiten am Behandlungsort unterschiedlich stark auslenkbar ist.

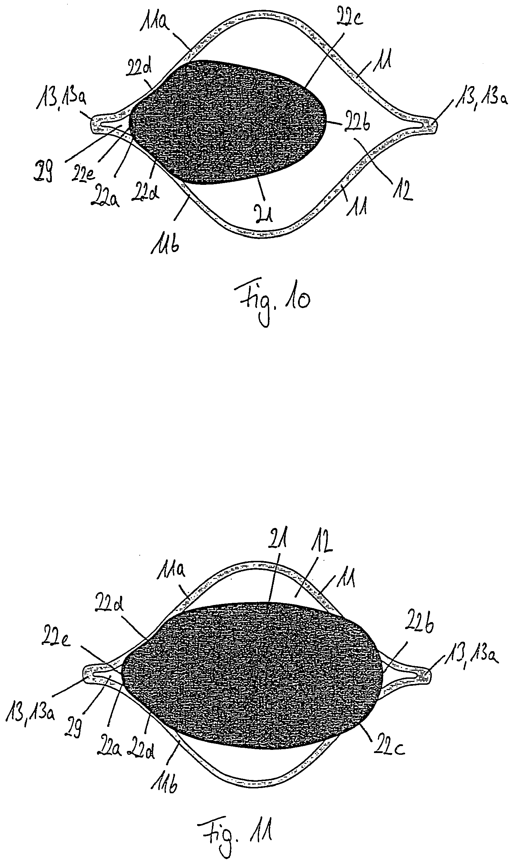

Die Klappe 21 kann unterschiedliche Geometrien aufweisen, wie nachfolgend anhand der Fig. 10 bis 14 erläutert wird:
In den Fig. 10 bis 14 ist jeweils eine einzige Zelle 12 einer medizinischen Vorrichtung nach einem bevorzugten Ausführungsbeispiel gezeigt. Im Allgemeinen kann die medizinische Vorrichtung wenigstens eine Zelle 12 aufweisen, die mit einer Klappe 21 nach einem der Ausführungsbeispiele gemäß den Fig. 10 bis 14 ausgebildet ist. Es ist auch möglich, dass die medizinische Vorrichtung mehrere Zellen 12 aufweist, die unterschiedlich ausgebildete Klappen 21 umfassen. Mit anderen Worten kann die medizinische Vorrichtung bzw. die Wandung 10 eine beliebige Kombination aus Zellen nach den Fig. 10 bis 14 aufweisen. Vorzugsweise weist die Wandung 10 mehrere gleichartige Zellen auf. Insbesondere ist vorgesehen, dass die in den Fig. 10 bis 14 einzeln dargestellten Klappen 21 in einer rohrförmigen Wandung 10 mehrfach vorliegen und miteinander verbunden sind. Die in der Wandung 10 mehrfach vorliegenden und zueinander benachbarten Klappen 21 sind vorzugsweise derart miteinander verbunden, dass die Klappen 21 gemeinsam eine einzige Membran 20 bilden. Umgekehrt weist die Wandung 10 vorzugsweise eine einzige Membran auf, die mehrere Klappen 21 bildet. Dabei können die Klappen 21 in unmittelbar benachbarten Zellen 12 angeordnet sein. Es ist auch möglich, dass die Klappen 21 derart verteilt über die Wandung 10 der medizinischen Vorrichtung angeordnet sind, dass zumindest zwischen einzelnen Klappen 21 freie Zellen 12 angeordnet sind.

Die Membran ist mit der Trägerstruktur bzw. den Stegen 11 im Wesentlichen punktuell verbunden. Die punktuelle Verbindung zwischen der Membran 20 und den Stegen 11 erfolgt vorzugsweise an den Stellen, die jeweils das erste Ende 22a einer Klappe 21 bilden. In Bezug auf die Stege 11 erfolgt die Verbindung der Membran 20 im Wesentlichen in der Nähe bzw. in der Nachbarschaft eines Knotenpunkts 13. Vorzugsweise ist die Membran 20 bzw. die Klappe 21 an den Stellen der Stege bzw. der Wandung 10 verbunden, die beim Übergang der medizinischen Vorrichtung vom komprimierten Zustand in den expandierten Zustand eine vergleichsweise kleine Bewegung vollziehen. Besonders bevorzugt ist dabei die Verbindung der Klappe 21, insbesondere des ersten Endes 22a, direkt im Knotenbereich 13, der sich bei der Expansion der medizinischen Vorrichtung im Wesentlichen nicht verformt. Zumindest ist die Klappe 21 zwischen dem ersten Steg 11a und dem zweiten Steg 11b in der Nähe des Knotenpunkts 13 angeordnet. Dabei schließt der erste Steg 11a und der zweite Steg 11b einen spitzen Winkel ein. Bei der Expansion der rohrförmigen Wandung 10 erfolgt im Bereich des spitzen Winkels zwischen dem ersten Steg 11a und dem zweiten Steg 11b eine vergleichsweise kleine Relativbewegung. Besonders bevorzugt ist, wenn das erste Ende 22a der Klappe 21, wie in den Fig. 10 bis 14 dargestellt, an einem Bereich der Stege 11 befestigt ist, der in Richtung der Klappe 21 bzw. in Richtung des Zentrums der Zelle 12 gekrümmt ist. Dadurch wird erreicht, dass bei der Komprimierung bzw. Crimpung der medizinischen Vorrichtung veranlasst wird, dass sich die Klappe 21 faltet. Eine Dehnung, also die Erzeugung einer Spannung innerhalb der Klappe 21 bei der Komprimierung der medizinischen Vorrichtung wird somit vermieden.

Die Geometrie der Zelle 12 und der Klappe 21 gemäß den Fig. 10 und 11 entspricht im Wesentlichen der Geometrie der Zellen 12 und der Klappen 21 gemäß Fig. 1 und 2. Ein Unterschied zu den Ausführungsbeispielen gemäß Fig. 1 und 2 besteht darin, dass die blattartige Form der Klappe 21 in Richtung des zweiten Endes 22b eine flachere freie Kante 22c umfasst. Die freie Kante 22c gemäß Fig. 10 ist im Wesentlichen parabelförmig ausgebildet. Das bedeutet, dass die freie Kante 22c ausgehend vom ersten Steg 11a bis zum zweiten Steg 11b im Wesentlichen eine einheitliche Krümmung im Uhrzeigersinn aufweist, wobei der Krümmungsradius variiert. Dasselbe gilt im Wesentlichen für das Ausführungsbeispiel gemäß Fig. 11, wobei ein vorderer Abschnitt der freien Kante 22c im Bereich des zweiten Endes 22b im Vergleich zum Ausführungsbeispiel gemäß Fig. 10 flacher ausgebildet ist. Ferner sind die seitlichen Abschnitte der freien Kante 22c, die sich zwischen dem zweiten, freien Ende 22b der Klappe 21 und dem ersten und zweiten Steg 11a, 11b erstrecken, im Vergleich zu dem Ausführungsbeispiel gemäß Fig. 10 stärker nach außen gewölbt bzw. gekrümmt. Insgesamt unterscheiden sich die Ausführungsbeispiele gemäß Fig. 10 und 11 dadurch, dass sich die Klappe 21 gemäß Fig. 10 in die Zelle hinein erstreckt, wogegen die Klappe 21 gemäß Fig. 11 sich über die Zelle 12 hinaus erstreckt bzw. die Zelle 12 überlappt. Gemäß Fig. 10 ist das zweite Ende 22b der Klappe 21 vollständig innerhalb der Zelle 12 angeordnet. In Fig. 11 ist zu erkennen, dass das zweite Ende 22b der Klappe 21 über die weiteren Stege der Zelle 12 vorsteht. Die Klappe 21 liegt in der Schließstellung lose bzw. frei, insbesondere unverbunden, auf den weiteren Stegen 11 auf.

Bei den Ausführungsbeispielen gemäß Fig. 10, 11 und 14 weist die innere Kante 22e der Klappe 21 eine zum ersten Knotenpunkt 13a gerichtete Krümmung auf. Die Krümmung der inneren Kante 22e verläuft vom ersten Steg 11a zum zweiten Steg 11b gleichmäßig bzw. kontinuierlich im Gegenuhrzeigersinn. Ein Einschnitt in die Klappe 21 wird mit der inneren Kante 22e gemäß Fig. 10, 11 und 14 nicht gebildet. Die durch den ersten Steg 11a, den zweiten Steg 11b und die innere Kante 22e der Klappe 21 gebildete Faltöffnung 29 weist im Wesentlichen eine trichterartige Form auf.

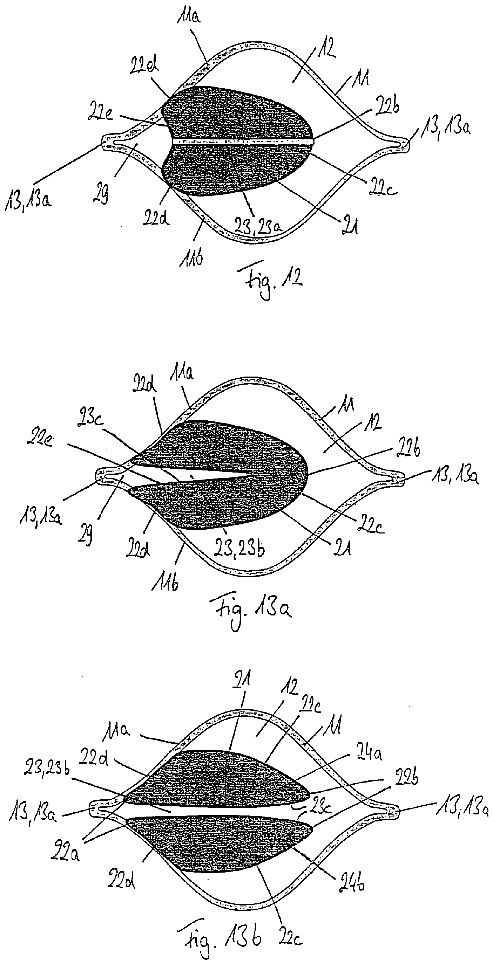

In Fig. 12 ist zu erkennen, dass die Klappe 21 eine Faltlinie 23 aufweisen kann, die sich im Wesentlichen in Längsrichtung der Klappe 21 erstreckt. Die Faltlinie 23 fluchtet mit zwei Knotenpunkten 13, die in Längsrichtung bzw. Axialrichtung der rohrförmigen Wandung 10 einander nachgeordnet bzw. zueinander fluchtend angeordnet sind. Insbesondere fluchtet die Faltlinie 23 mit zwei ersten Knotenpunkten 13a zweier in Längsrichtung der Wandung 10 unmittelbar benachbarter Zellen 12. Die Faltlinie 23 kann wie in Fig. 12 eine Nut 23a umfassen. Die Nut 23a ist vorzugsweise derart angepasst, dass eine Faltung der Klappe 21 beim Komprimieren der rohrförmigen Wandung 10 erleichtert ist. Insbesondere wird durch die Nut 23a eine definierte Faltkante vorgegeben, die im Wesentlichen als Drehachse dient, um die ein erster Klappenflügel 24a und zweiter Klappenflügel 24b der Klappe 21 zum Falten drehbar sind. Der erste Klappenflügel 24a und der zweite Klappenflügel 24b werden also bei der Komprimierung der rohrförmigen Wandung 10 entlang der Faltlinie 23, insbesondere der Nut 23a, übereinander geklappt.

Die Nut 23a kann beispielsweise durch ein Ätzverfahren hergestellt werden. Vorzugsweise weist die Nut 23a eine Tiefe auf, die wenigstens 25 %, insbesondere wenigstens 30%, insbesondere wenigstens 40 %, insbesondere wenigstens 50 %, insbesondere wenigstens 60 %, insbesondere wenigstens 70 %, insbesondere wenigstens 75 %, der Wandstärke der Klappe 21 entspricht. Es ist überdies möglich, dass mehrere Nuten 23a vorgesehen sind, die sich unterschiedlichen Orientierungen über die Klappe 21 erstrecken. Auf diese Weise kann das Falten der Klappe 21 in komplexen Geometrien ermöglicht werden. Die Nut 23a erstreckt sich geradlinig über die Klappe 21. Die Nut 23a kann sich auch in anderer Orientierung, insbesondere in wechselnder Orientierung über die Klappe 21 erstrecken. Vorteilhafterweise falten sich die Klappenflügel 24a, 24b bei der Komprimierung der rohrförmigen Wandung 10 radial nach innen, um die Anordnung der medizinischen Vorrichtung in einem Katheter 60 zu erleichtern. Es ist auch möglich, dass die Klappe 21 mehrere Faltlinien 23 aufweist, so dass eine Mehrfachfaltung erreicht wird. Die Mehrfachfaltung kann ziehharmonikaartig ausgebildet sein. Die Faltlinien 23 können sich auch teilweise in Umfangsrichtung der rohrförmigen Wandung 10 erstrecken. Generell können sich die Faltlinien 23 abschnittsweise über die Klappe 21 erstrecken. Die Faltlinie 23 kann ferner eine Rippe bzw. einen Vorsprung aufweisen, der eine Drehachse für die Faltung der Klappenflügel 24a, 24b bildet.

In Fig. 12 ist überdies eine weitere mögliche Gestaltung der Faltöffnung 29 dargestellt. Im Wesentlichen weist die Faltöffnung 29 eine drachenviereckige Form auf, wobei die durch die innere Kante 22e gebildeten kürzeren Kanten des Drachenvierecks eine Krümmung aufweisen. Das bedeutet, dass die durch die beiden kürzeren Kanten des Drachenvierecks gebildete Ecke, die im Schnittpunkt der Faltlinie 23 mit der inneren Kante 22e angeordnet ist, abgerundet ist. Im expandierten Zustand der rohrförmigen Wandung 10 ist das freie zweite Ende 22b der Klappe 21 gemäß Fig. 12 vollständig innerhalb der Zelle 12 angeordnet.

Bei den Ausführungsbeispielen gemäß Fig. 13a und 13b weist die Faltlinie 23 einen Spalt 23b auf, wobei der Spalt 23b gemäß Fig. 13a die Klappe 21 teilweise trennt. Der Spalt 23b entspricht also im Wesentlichen einer ausgeprägten Form des Einschnitts der in anderen Ausführungsbeispielen durch die innere Kante 22e der Klappe 21 gebildet ist. Insbesondere weist die innere Kante 22e gemäß Fig. 13a zwei gerade Spaltkanten 23c auf, die zum Zentrum der Zelle 12 hin konvergieren. Die Faltöffnung 29 ist dadurch im expandierten Zustand der rohrförmigen Wandung 10 bis in das Zentrum der Zelle 12 verlängert. Auf diese Weise wird erreicht, dass bei der Expansion der Wandung 10 der erste Klappenflügel 24a und der zweite Klappenflügel 24b relativ zueinander bewegbar sind, so dass bei der Expansion der Wandung 10 auf die Klappe 21 wirkende Spannungen vermieden werden. Insbesondere wird eine Dehnung der Klappe 21 in Umfangsrichtung der rohrförmigen Wandung 10 vermieden.

Wie in Fig. 13b dargestellt, kann der Spalt 23b die Klappe 21 vollständig in einen ersten Klappenflügel 24a und einen zweiten Klappenflügel 24b trennen. Die Zelle 12 weist also zwei separate Klappenflügel 24a, 24b auf, die jeweils mit einem einzigen Steg 11 verbunden sind. Die freien Enden der Klappenflügel 24a, 24b ragen im expandierten Zustand der rohrförmigen Wandung 10 in die Zelle 12 hinein. Es ist auch möglich, dass die freien Enden der Klappenflügel 24a, 24b die Zelle 12 überlappen, also über die weiteren Stege 11 im expandierten Zustand der Wandung 10 vorstehen.

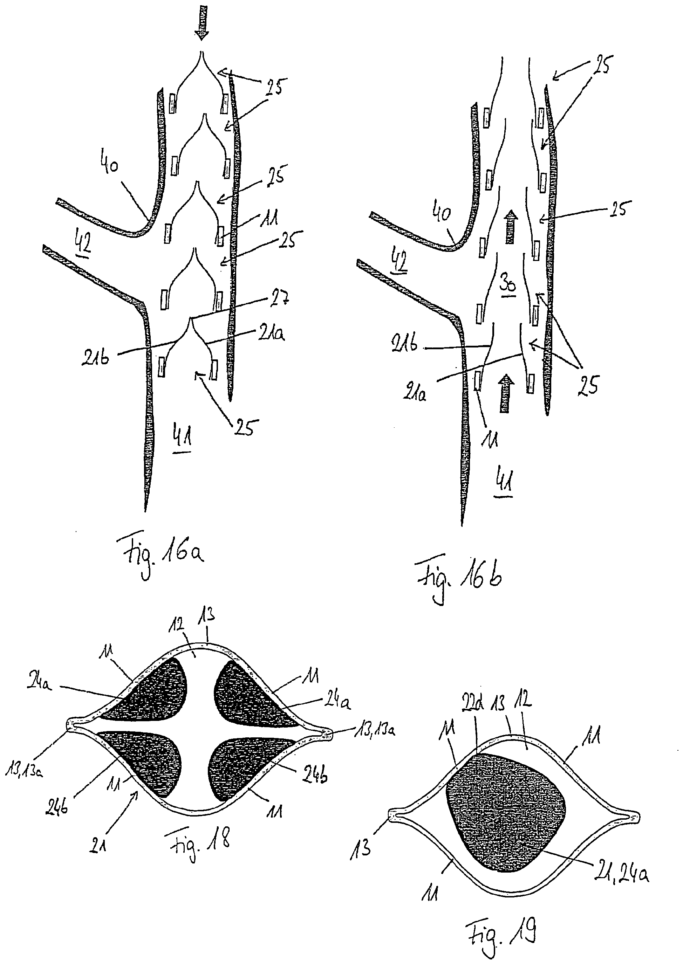

Ein ähnliches Ausführungsbeispiel ist in Fig. 18 dargestellt, wobei insgesamt vier Klappenflügel 24a, 24b eine Klappe 21 bilden. Die vier Klappenflügel sind jeweils an einem Steg 11 der Zelle 12 angeordnet. Die Verbindung zwischen den Klappenflügeln 24a, 24b mit den Stegen 11 erfolgt linienförmig. Das bedeutet, dass die Klappenflügel 24a, 24b jeweils eine Verbindungslinie aufweisen, die sich entlang des zugehörigen Stegs 11 erstreckt.

Für alle Ausführungsbeispiele gilt generell, dass die Klappe 21 mit dem Steg 11 linienförmig verbunden ist. Ferner gilt allgemein, dass eine Verbindung der Klappe 21 mit einem vergleichsweise geraden bzw. geradlinig ausgebildeten Abschnitt des Stegs 11 bevorzugt ist. Damit wird vermieden, dass beim Komprimieren der rohrförmigen Wandung 10 auf die Klappe 21 Kräfte wirken, die ein Falten oder ein Dehnen der Klappe 21 verursachen. Dies wird beispielsweise bei dem Ausführungsbeispiel gemäß Fig. 18 dadurch erreicht, dass die Klappenflügel 24a, 24b jeweils mit einem mittleren Bereich des zugehörigen Stegs 11 verbunden sind. Insbesondere erfolgt die Verbindung zwischen den Klappenflügel 24a, 24b und dem Steg 11 in einem vergleichsweise geradlinigen Bereich in der Mitte des S-förmig gestalteten Stegs 11. Bei den weiteren Ausführungsbeispielen gemäß Figuren 10 bis 14 wird die Entlastung der Klappe 21 beim Komprimieren der rohrförmigen Wandung 10 beispielsweise dadurch erreicht, dass entweder eine Faltöffnung 29 oder ein Spalt 23b vorgesehen ist. Dadurch wird eine Verbindung der Klappe 21 bzw. der Klappenflügel 24a, 24b in Bereichen der Stege 11, die sich bei dem Übergang der rohrförmigen Wandung 10 vom komprimierten in den expandierten Zustand und umgekehrt stark verformen, vermieden.

Ein weiteres Ausführungsbeispiel, bei dem die Klappe 21 in einem geradlinigen Bereich des Stegs 11 angelenkt bzw. mit dem Steg 11 verbunden ist, zeigt beispielhaft Fig. 19. Dabei ist die Klappe 21 durch einen einzigen Klappenflügel 24a gebildet, der einen Halteabschnitt 22d aufweist, der linienförmig mit dem Steg 11, insbesondere einem geradlinigen Bereich des Stegs 11, verbunden ist. Alternativ ist auch eine punktuelle Verbindung möglich. Für alle Ausführungsbeispiele, bei denen die Verbindung zwischen der Klappe 21 und dem Steg 11 in einem geradlinigen Bereich des Stegs 11 erfolgt, ist vorteilhaft vorgesehen, dass die Länge des geradlinigen Bereichs des Stegs 11 in Bezug auf die Gesamtlänge des Stegs 11, also dem Abstand zwischen zwei durch einen S-förmigen Steg 11 verbundenen Knotenpunkten 13, wenigstens 10%, insbesondere wenigstens 20%, insbesondere wenigstens 30%, insbesondere wenigstens 40%, insbesondere wenigstens 50%, insbesondere wenigstens 60%, insbesondere wenigstens 70%, insbesondere wenigstens 80%, insbesondere wenigstens 90%, der Steglänge entspricht.

Im Allgemeinen kann die Steglänge dem Stegverlauf folgend gemessen werden. Das bedeutet, dass die Steglänge der Wegstrecke zwischen zwei Knotenpunkten 13 entlang des Stegs 11 entspricht. Alternativ kann die Steglänge auch dem direkten Abstand zwischen zwei Knotenpunkten 13 entsprechen, also als Luftlinie zwischen zwei Knotenpunkten 13 betrachtet werden.

Besonders bevorzugt ist vorgesehen, dass sich der Halteabschnitt 22d über die gesamte Länge des geradlinigen Bereichs des Stegs 11 erstreckt. Insbesondere kann der Halteabschnitt 22d über die gesamte Länge des geradlinigen Bereichs des Stegs 11 linienförmig mit dem Steg 11 verbunden sein. Der Halteabschnitt 22d weist analog zum geradlinigen Bereich des Stegs 11 eine Länge auf, die wenigstens 10%, insbesondere wenigstens 20%, insbesondere wenigstens 30%, insbesondere wenigstens 40%, insbesondere wenigstens 50%, insbesondere wenigstens 60%, insbesondere wenigstens 70%, insbesondere wenigstens 80%, insbesondere wenigstens 90%, der gesamten Steglänge entspricht. Es ist nicht ausgeschlossen, dass sich der Halteabschnitt 22d über den geradlinigen Bereich des Stegs 11 hinaus erstreckt. Der Halteabschnitt 22d kann sich beispielsweise in die gekrümmten Abschnitte des Stegs 11 erstrecken. Ferner kann der Halteabschnitt 22d teilweise bzw. abschnittsweise mit dem geradlinigen Abschnitt des Stegs 11 verbunden sein. Der Halteabschnitt 22d kann sich insgesamt über einen Teil des geradlinigen Abschnitts des Stegs 11 erstrecken.

Bei dem Ausführungsbeispiel gemäß Fig. 19 weist die Klappe 21 einen einzigen Klappenflügel 24a auf, der einen Halteabschnitt 22d umfasst, wobei der Halteabschnitt 22d mit dem geradlinigen Bereich des Stegs 11 verbunden ist. Wenn über den Umfang der Wandung 10 mehrere Klappen 21 vorgesehen sind, ist es bevorzugt, dass die Klappen 21 gleichartig orientiert sind. Die Klappen 21, insbesondere benachbarte Klappen 21 weisen also im Wesentlichen dieselbe Längsorientierung auf. Auf diese Weise wird sichergestellt, dass sich die Klappen 21 beim Übergang der Wandung 10 in den komprimierten Zustand überlappen, also auf dem Umfang der Wandung 10 im Wesentlichen bündig aufliegend gleiten. Eine Ausweichbewegung der Klappen 21 in radialer Richtung bezogen auf die rohrförmige Wandung 10 wird somit vermieden. Es ist auch möglich, dass die Klappe 21 derart angepasst ist, dass sich die Klappe 21 beim Komprimieren der Wandung 10 in die Zelle 12 hinein oder radial nach außen bezogen auf die rohrförmige Wandung 10 faltet bzw. allgemein verformt.

Anhand eines weiteren Ausführungsbeispiels zeigt Fig. 14, dass die Klappe 21 eine Strukturierung umfassen kann. Insbesondere ist vorgesehen, dass die Klappe 21 eine Porenstruktur 28 oder Perforierung aufweist. Allgemein ist die Porenstruktur oder Perforierung durch Öffnungen in der Membran 20 gebildet, die in einem Muster angeordnet sind. Alternativ kann die Klappe 21, insbesondere eine Oberfläche der Klappe 21 eine Rillenstruktur oder eine Fleecestruktur aufweisen. Im Allgemeinen fördert die Strukturierung der Klappe 21 die Endothelialisierung. Die Strukturierung ist insbesondere für Klappen 21 vorgesehen, die auf dem Außenumfang 14a der Wandung 10 angeordnet sind. Die Strukturierung ist dabei auf einer radial äußeren Fläche der Klappe 21 angeordnet. Vorzugsweise sind zumindest die Klappen 21 strukturiert, die im Gebrauch in Kontakt mit einer Gefäßwand 40 stehen. In der Strukturierung können Medikamente eingelagert sein.

Fig. 15 zeigt einen Ausschnitt einer rohrförmigen Wandung 10 einer medizinischen Vorrichtung nach einem weiteren bevorzugten Ausführungsbeispiel, wobei mehrere Zellen 12 gezeigt sind, die jeweils eine Klappe 21 aufweisen. Die Klappen 21 sind zumindest an der Oberfläche strukturiert. Die Strukturierung kann auch eine Porenstruktur 28 oder Perforation umfassen. Das bedeutet, dass die Klappe 21 durchgehend bzw. durchgängig strukturiert, insbesondere durch Öffnungen durchbrochen sein kann. Die Zellen 12 bzw. die die Zellen 12 bildenden Stege 11 sind in Knotenpunkten 13 miteinander verbunden. In den Knotenpunkten 13 sind die Klappen 21 jeweils mit den Stegen verbunden. Die ersten Enden 22a der Klappen 21 sind also bei dem Ausführungsbeispiel gemäß Fig. 15 unmittelbar im Bereich bzw. im Knotenpunkt 13 mit den Stegen 11 gekoppelt. Das bedeutet, dass bei dem Ausführungsbeispiel gemäß Fig. 15 keine Faltöffnung 29 vorgesehen ist. Die Klappen 21 überlappen jeweils die zugehörige Zelle 12. Das bedeutet, dass jeweils das zweite Ende 22b der Klappe 21 über die dem ersten Ende 22a gegenüber angeordneten Steg 11 der Zelle 12 vorsteht.

Im Hinblick auf die Funktionsweise beim Übergang vom expandierten Zustand in den komprimierten Zustand der Wandung 10 unterscheidet sich das Ausführungsbeispiel gemäß Fig. 15 von den übrigen Ausführungsbeispielen. Insbesondere ist bei der medizinischen Vorrichtung gemäß Fig. 15 vorgesehen, dass sich die Klappen 21 im komprimierten Zustand dachziegelartig überlappen. Da die Klappen 21 punktuell mit dem jeweils ersten Ende 22a in den Knotenpunkt 13 mit der tragenden Struktur, nämlich den Stegen 11, verbunden sind, ist gewährleistet, dass die Wandung 10 im komprimierten Zustand einen relativ kleinen Querschnittsdurchmesser einnimmt.

Insbesondere wird bei der Komprimierung der rohrförmigen Wandung 10 eine Verformung, insbesondere eine Faltung der Klappe 21 in die Zelle 12 oder eine Dehnung der Klappe 21, vermieden. Die Klappe 21 gleitet bei der Komprimierung auf der rohrförmigen Wandung 10.

Die rohrförmige Wandung 10 gemäß Fig. 15 umfasst wenigstens eine erste Reihe 12a von Zellen 12 und eine zweite Reihe 12b von Zellen 12. Die erste und zweite Reihe 12a, 12b erstreckt sich jeweils in Umfangsrichtung über die Wandung 10. Die erste Reihe 12a und die zweite Reihe 12b sind in Längsrichtung der Wandung 10 versetzt angeordnet. Das bedeutet, dass die ersten Knotenpunkte 13a der zweiten Reihe 12b in Umfangsrichtung mit den zweiten Knotenpunkten 13b der zweiten Reihe 12b fluchten bzw. identisch sind. Insbesondere bilden die zweiten Knotenpunkte 13b der ersten Reihe 12a gleichzeitig die ersten Knotenpunkte 13a der zweiten Reihe 12b. Im komprimierten Zustand der Wandung 10 überlappen sich die Klappen 21 der ersten und zweiten Reihe 12a, 12b jeweils in Umfangsrichtung. Das bedeutet, dass eine erste Klappe 21a der ersten Reihe 12a in Umfangsrichtung eine weitere erste Klappe 21a der ersten Reihe 12a überlappt. Gleichzeitig überlappt eine zweite Klappe 21b der zweiten Reihe 12b eine weitere zweite Klappe 21b der zweiten Reihe 12b in Umfangsrichtung.

Die ersten Klappen 21a der ersten Reihe 12a sind im expandierten Zustand der Wandung 10 in Umfangsrichtung der Wandung 10 zu den zweiten Klappen 21b der zweiten Reihe 12b versetzt angeordnet. Daher überlappen die ersten Klappen 21a der ersten Reihe 12a im komprimierten Zustand der Wandung 10 jeweils zwei zweite Klappen 21b der zweiten Reihe 12b. Es ist auch möglich, dass die erste Klappe 21a der ersten Reihe 12a eine zweite Klappe 21b der zweiten Reihe 12b überlappt und gleichzeitig in eine in Umfangsrichtung benachbart zur zweiten Klappe 21b angeordnete weitere zweite Klappe 21b der zweiten Reihe 12b die erste Klappe 21a der ersten Reihe 12a überlappt. Insgesamt ist also vorgesehen, dass sich die Klappen 21 in Umfangsrichtung der Wandung 10 dachziegelartig überlappen.

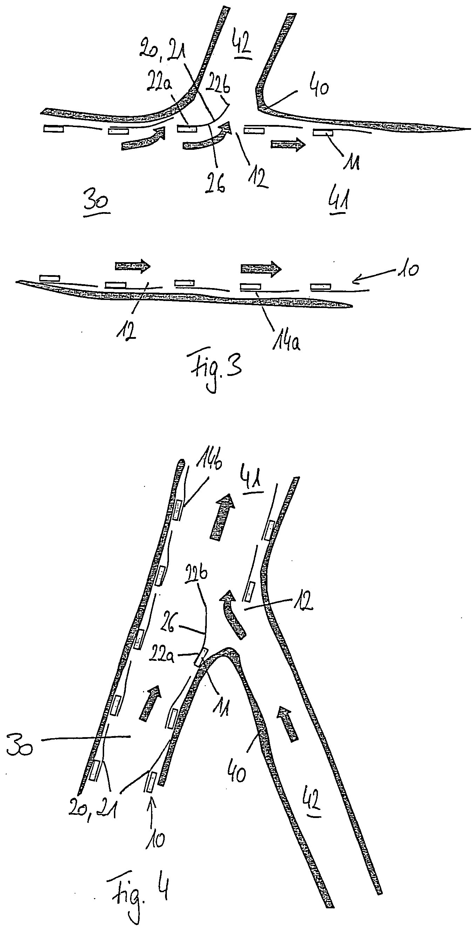

Nachfolgend werden besondere, bevorzugte Anwendungsmöglichkeiten der medizinischen Vorrichtung beschrieben, wobei auf die Fig. 3 bis 9 Bezug genommen wird:
Zweckmäßigerweise wird durch die flexible Klappe eine Ventilfunktion erreicht. Im Allgemeinen ist die tragende Struktur aus Stegen 11 mit einer Membran versehen, die in radialer Richtung bezüglich der Längsachse der rohrförmigen Wandung 10 bzw. allgemein der medizinischen Vorrichtung ventilartige Elemente aufweist. Die ventilartigen Elemente sind durch die Klappen 21 gebildet. Besonders vorteilhaft ist die Ventilfunktion im Bereich von Blutgefäßen einsetzbar. Dabei ist die rohrförmige Wandung 10 als Gitterstruktur eines Stents oder Flow Diverters ausgebildet. Durch die ventilartig betätigbaren Klappen 21 wird ein Blutfluss in radialer Richtung durch die rohrförmige Wandung 10 ermöglicht. Das kann im Bereich von Perforationen, vorzugsweise innerhalb von Aneurysmen, und/oder Bifurkationen bzw. allgemein Nebengefäßen 42 vorteilhaft sein. Die Klappe 21 kann im Allgemeinen derart angepasst sein, dass die Zelle 12 zur Durchströmung freigegeben wird, wenn sich zwischen den axialen Hohlkanal 30 der medizinischen Vorrichtung und einem Raum außerhalb der rohrförmigen Wandung 10 ein Druckgradient einstellt. Ist die Druckdifferenz zwischen dem axialen Hohlkanal 30 und dem Bereich außerhalb der rohrförmigen Wandung 10 vergleichsweise klein oder gleich null, bildet die geschlossene Klappe 21 einen Schutz vor Partikelablösung, beispielsweise im Bereich von vulnerablen Plaques. In der Schließstellung kann die Klappe 21 auch als Flow Diverter dienen. Beispielsweise nimmt die Klappe 21 die Schließstellung ein, wenn nach erfolgreicher Behandlung eines Aneurysmas 50 die Blutströmung innerhalb des Aneurysmas 50 gestoppt ist. Dasselbe gilt, wenn die Klappe 21 direkt an einer Gefäßwand 40 anliegt.

In den Fig. 3 bis 9 ist die Strömungsrichtung der Fluidströmung bzw. konkret der Blutströmung durch Pfeile angedeutet. Dabei zeigen die Fig. 3 bis 5 die vorteilhafte Anwendung der medizinischen Vorrichtung im Bereich von Bifurkationen, also Gefäßverzweigungen. Die Gefäßverzweigung weist ein Hauptgefäß 41 und ein Nebengefäß 42 auf. Bei dem Ausführungsbeispiel gemäß Fig. 3 strömt Körperfluid vom Hauptgefäß 41 in das Nebengefäß 42. Vom Hauptgefäß 41 wird also ein Teil der Fluidströmung in das Nebengefäß 42 abgezweigt. Dabei ist es besonders vorteilhaft, wenn die in diesem Bereich eingesetzte medizinische Vorrichtung Klappen 21 aufweist, die auf dem Außenumfang 14a der Wandung 10 angeordnet sind. Die Klappen 21 werden vorzugsweise derart ausgerichtet, dass die freien zweiten Enden 22b der Klappen 21 in Richtung der Hauptfluidströmung zeigen. Im Bereich der Abzweigung zum Nebengefäß 42 bewirkt die Fluidströmung die Überführung der Klappen 21 von der Schließstellung in die Offenstellung, so dass Körperfluid vom Hauptgefäß 41 in das Nebengefäß 42 strömen kann. Dazu wird das zweite Ende 22b der Klappe 21 in der Offenstellung radial nach außen ausgelenkt. Es ist auch möglich, dass das freie zweite Ende 22b radial nach innen ausgelenkt wird, um beispielsweise eine Rückströmung aus dem Nebengefäß 42 in das Hauptgefäß 41 zu ermöglichen. Vorzugsweise wird eine derartige Rückströmung blockiert, indem die Klappe 21 derart angepasst ist, dass eine Auslenkung in nur eine Richtung möglich ist. Beispielsweise kann die Klappe 21 die Zelle 12 überlappen, so dass das zweite Ende 22b in der Schließstellung auf den Stegen 11 der Zelle 12 aufliegt. Eine bezüglich der rohrförmigen Wandung 10 radial nach innen gerichtete Auslenkung des zweiten Endes 22b wird somit verhindert. Dabei ist nicht ausgeschlossen, dass die einseitige Ventilfunktion erreicht wird, wenn die Klappe 21 in beide Richtungen auslenkbar ist. Die einseitige Ventilfunktion kann beispielsweise auch durch die physiologischen Strömungsverhältnisse erreicht werden.

Bei dem Ausführungsbeispiel gemäß Fig. 4 sind die Klappen 21 auf dem Innenumfang der rohrförmigen Wandung 10 angeordnet. Diese Ausführungsform eignet sich besonders für die Stützung von Gefäßen im Bereich von Gefäßverzweigungen, bei denen eine Fluidströmung aus einem Nebengefäß 42 in ein Hauptgefäß 41 mündet. Die Fluidströmung aus dem Nebengefäß 42 trifft also von radial außen auf die medizinische Vorrichtung und soll in den Hohlkanal 30 geleitet werden. Die auf dem Innenumfang 14b angeordneten Klappen ermöglichen eine einfache Auslenkung der zweiten Enden 22b nach radial innen, so dass die Fluidströmung aus dem Nebengefäß 42 in das Hauptgefäß 41 gelangen kann. Es ist auch möglich, dass die Klappen 21 auf dem Außenumfang 14a der rohrförmigen Wandung 10 angeordnet sind und in der Offenstellung nach innen ausgelenkt werden. Analog zu der Konfiguration gemäß Fig. 3 ist es möglich, dass eine Auslenkung der Klappen 21 in eine Richtung blockiert ist, beispielsweise indem die Klappe 21 die zugehörige Zelle 12 überlappt. Wie in Fig. 4 gut zu erkennen, sind die Stege 11 auf dem Außenumfang der rohrförmigen Wandung 10 angeordnet. Dadurch ergibt sich eine Oberflächenstruktur auf dem Außenumfang 14a, der eine Endothelialisierung der gesamten medizinischen Vorrichtung begünstigt. Die Stege 11 können auch als Halteelemente dienen, die in die Gefäßwand 40 eingreifen.

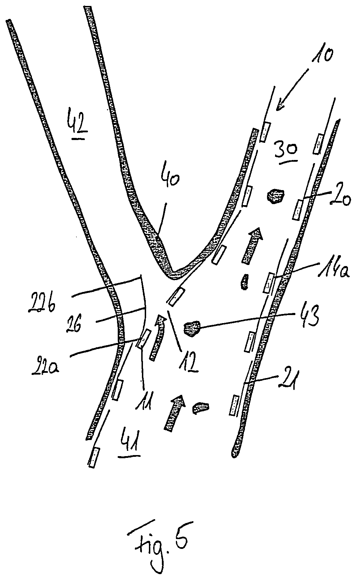

Das Ausführungsbeispiel gemäß Fig. 5 entspricht im Wesentlichen dem Ausführungsbeispiel gemäß Fig. 3, wobei die Klappen 21 auf dem Außenumfang 14a der Wandung 10 angeordnet sind und eine Fluidströmung von einem Hauptgefäß 41 in ein Nebengefäß 42 zulassen. In Fig. 5 ist ferner dargestellt, dass die medizinische Vorrichtung vorteilhaft dazu eingesetzt werden kann, Partikel in der Fluidströmung, beispielsweise Thrombusbestandteile in ein bestimmtes Gefäß zu leiten. Durch die Abdeckung des Nebengefäßes 42 mit der flexiblen Membran 20, insbesondere den Klappen 21, wird erreicht, dass die in der Fluidströmung geführten Partikel 43 durch den axialen Hohlkanal 30 geleitet werden. Ein Abfluss von Partikeln 43 in das Nebengefäß 42 wird vermieden.

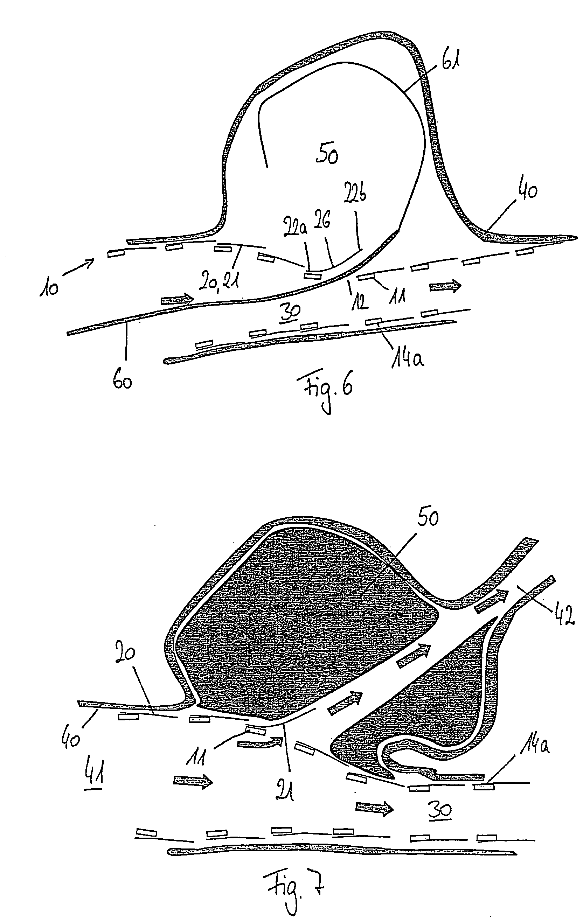

Eine weitere bevorzugte Anwendung der medizinischen Vorrichtung ist die Behandlung von Aneurysmen 50, wie beispielhaft in den Fig. 6 und 7 dargestellt.

Dazu ist vorteilhaft vorgesehen, dass zumindest der Bereich der rohrförmigen Wandung 10, der dem Aneurysma 50 zugewandt ist, mit der flexiblen Membran 20 versehen ist. Die flexible Membran 20 bzw. die Klappen 21 decken die Öffnung des Aneurysmas 50 ab, so dass eine Blutströmung in das Aneurysma 50 hinein unterbunden wird. Dies entspricht im Wesentlichen der Funktion bekannter Flow Diverter. Überdies ermöglicht die flexible Membran 20, insbesondere die Klappen 21 die Einführung eines Katheters 60 durch die Zelle 12 in das Aneurysma 50, wobei durch die Vorschubkraft des Katheters 60 die Klappe 21 von der Schließstellung in die Offenstellung überführt wird. Auf diese Weise wird ermöglicht, das Aneurysma 50 zusätzlich mit einem Coil 61 zu füllen, um eine kurzfristige Embolisierung zu erreichen.

Besonders zweckmäßig ist der Einsatz der medizinischen Vorrichtung bei Aneurysmen im Bereich von Gefäßverzweigungen bzw. Bifurkationen, wie in Fig. 7 dargestellt. Bei derartigen Bifurkationsaneurysmen kann die flexible Membran 20 eine Doppelfunktion übernehmen. Ein Teil der Membran 20, insbesondere ein Teil der Klappen 21, der im Bereich des Aneurysmas 50 angeordnet ist, verschließt vorteilhafterweise den Zustrom von Blut in das Aneurysma 50. Gleichzeitig kann wenigstens eine Klappe 21 eine Blutströmung vom Hauptgefäß 41 in das Nebengefäß 42 sicherstellen, wobei eine Strömungspassage 44 gebildet wird, die sich durch das Aneurysma 50 erstreckt.

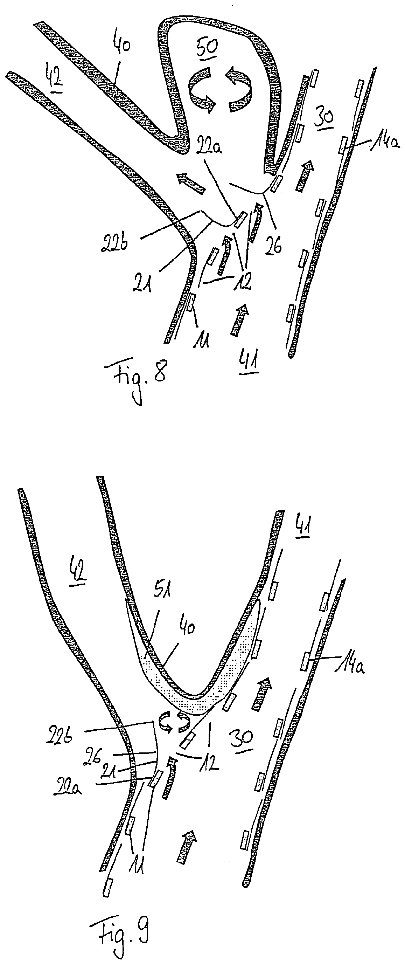

Diese vorteilhafte Eigenschaft wird auch bei dem Ausführungsbeispiel gemäß Fig. 8 deutlich. Dabei entspricht die medizinische Vorrichtung gemäß Fig. 8 in ihrem konstruktiven Aufbau im Wesentlichen dem Ausführungsbeispiel gemäß Fig. 3, wobei die medizinische Vorrichtung gemäß Fig. 8 entgegen der Strömungsrichtung in das Gefäß eingesetzt ist. Das bedeutet, dass die zweiten, freien Enden 22b der Klappen 21 entgegen der Strömungsrichtung der Fluidströmung zeigen. Wie in Fig. 8 durch die Pfeile dargestellt, wird auf diese Weise bewirkt, dass durch die Klappen 21 im Bereich der Abzweigung Körperfluid vom Hauptgefäß 41 in das Nebengefäß 42 abströmen kann. In der Offenstellung verhindert die Klappe 21 in der in Fig. 8 gezeigten Konfiguration ein direktes Einströmen des Körperfluids in das Aneurysma 50.

In Fig. 9 ist die vorteilhafte Behandlung einer Stenose im Bereich einer Bifurkation mit Hilfe der medizinischen Vorrichtung gezeigt. Dabei ermöglichen die in Strömungsrichtung ausgerichteten Klappen 21 eine abzweigende Fluidströmung vom Hauptgefäß 41 in das Nebengefäß 42. Zwischen dem Hauptgefäß 41 und dem Nebengefäß 42 ist in einer gemeinsamen Gefäßwand 40 eine Stenose ausgebildet. Durch die Klappe 21 kann eine Rückströmung des Körperfluids vom Nebengefäß 42 in das Hauptgefäß 41 verhindert werden. Dadurch wird vermieden, dass Stenosepartikel von der Stenose 51 in das Hauptgefäß 41 gelangen.

In den Fig. 16a und 16b ist eine vorteilhafte Ausgestaltung des nebengeordneten Aspekts der Erfindung dargestellt, bei dem die Klappen 21 derart angeordnet sind, dass sie in der Schließstellung den axialen Hohlkanal 30 verschließen, wie in Fig. 16a gezeigt. Die rohrförmige Wandung 10 der medizinischen Vorrichtung umfasst eine Stützstruktur aus Stegen 11, die die Form des Hohlkanals 30 vorgeben. Auf dem Innenumfang 14b der Wandung 10 sind wenigstens zwei flexible Membrane 20 angeordnet, die in axialer Richtung des Hohlkanals 30 zueinander beabstandet angeordnet sind. Die flexiblen Membrane 20 bilden jeweils eine Mehrfachklappe 25, die bei dem Ausführungsbeispiel gemäß Fig. 16a und 16b jeweils zwei Klappen 21 umfassen. Gemäß Fig. 16a und 16b sind jeweils fünf Membrane 20 vorgesehen, die jeweils eine Mehrfachklappe 25 mit zwei Klappen 21 bilden. Die Mehrfachklappe 25 kann auch mehr als zwei, insbesondere drei, Klappen 21 umfassen. Die Klappen 21 weisen jeweils eine Krümmung auf, die im Querschnitt S-förmig verläuft. Die freien zweiten Enden 22b der Klappen 21 einer Mehrfachklappe 25 berühren sich in der Schließstellung. Die Mehrfachklappe 25 bildet somit im Wesentlichen ein tropfenförmiges Längsschnittprofil. Die zweiten Enden 22b der Klappe 21 bilden in der Schließstellung eine Spitze 27 der Mehrfachklappe 25. Vorzugsweise ist die medizinische Vorrichtung im Körperhohlgefäß derart ausgerichtet, dass die Spitze 27 in Hauptströmungsrichtung weist. Dadurch wird erreicht, dass sich die Mehrfachklappe 25 selbsttätig öffnet, wenn eine Fluidströmung aus der Hauptströmungsrichtung wirkt. Hingegen schließt die Mehrfachklappe 25 selbsttätig, wenn eine Fluidströmung entgegen der Hauptströmungsrichtung entsteht. Auf diese Weise kann ein Rückfluss von Körperfluid vermieden werden.

Die medizinische Vorrichtung gemäß dem nebengeordneten Aspekt der Erfindung kann als Venenklappenprothese eingesetzt werden. Fig. 16a zeigt dabei die Mehrfachklappen 25 in der Schließstellung, wobei durch den Pfeil angedeutet ist, dass eine Rückströmung eines Körperfluids, insbesondere Blut, zum Verschluss der Mehrfachklappen 25 führt. Die Offenstellung der Mehrfachklappen 25 ist in Fig. 16b dargestellt, wobei die Pfeile die Fluidströmung in Hauptströmungsrichtung symbolisieren. In der Offenstellung erstrecken sich die Klappen 21 der Mehrfachklappe 25 im Wesentlichen auf dem Innenumfang 14b der Wandung 10.

Es ist auch möglich, dass bei den Ausführungsbeispielen gemäß Fig. 16a und 16b die Klappen 21 derart angepasst sind, dass das Gefäß vollständig verschlossen ist. Beispielsweise können die Klappen 21 eine Rückstellkraft aufweisen, die ein Öffnen der Mehrfachklappe 25 in Folge der Fluidströmung verhindert.

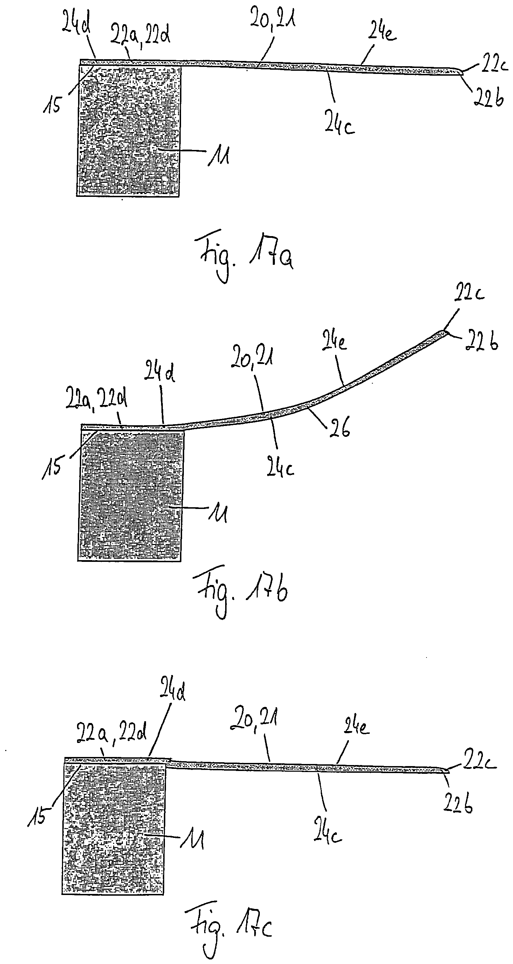

In den Fig. 17a bis 17c ist jeweils eine bevorzugte Anordnung der Klappe 21 auf einem Steg 11 dargestellt. In der Schließstellung erstreckt sich die Klappe 21 entlang der Wandung 10. Das bedeutet, dass die Klappe 21 im Wesentlichen parallel zu einer Außenkante 15 des Stegs 11 ausgerichtet ist. in der Offenstellung, wie in Fig. 17b dargestellt, ist das zweite Ende 22b der Klappe 21 gegenüber dem ersten Ende 22a ausgelenkt. Die Auslenkung erfolgt in radialer Richtung bezüglich der rohrförmigen Wandung 10. Durch die Auslenkung bildet die Klappe 21 eine Krümmung 26. Die Krümmung 26 kann von der Stärke der Fluidströmung abhängig sein, die durch die Zelle 12 hindurchtritt bzw. durch die Klappe 21 freigegeben ist.

Im Zusammenhang mit dem nebengeordneten Aspekt der Erfindung entspricht die Fig. 17a der Offenstellung und die Fig. 17b der Schließstellung eines bevorzugten Ausführungsbeispiels.

Im Allgemeinen kann die Klappe 21 einen freien Abschnitt 24c und einen fixierten Abschnitt 24d aufweisen. Der fixierte Abschnitt 24d ist fest mit dem Steg 11 verbunden. Der freie Abschnitt 24c erstreckt sich über die Zelle 12 und bildet im ausgelenkten Zustand des zweiten Endes 22b die Krümmung 26. Wie in Fig. 17c dargestellt, kann der freie Abschnitt 24c gegenüber dem fixierten Abschnitt 24d versetzt sein. Das bedeutet, dass sich zwischen dem fixierten Abschnitt 24d und dem freien Abschnitt 24c eine Stufe 24e bildet. Vorzugsweise ist der freie Abschnitt 24c in die Wandung 10 hineinversetzt. Das bedeutet, dass der freie Abschnitt 24c ausgehend vom Außenumfang 14a in Richtung des Innenumfangs 14b und umgekehrt ausgehend vom Innenumfang 14b in Richtung des Außenumfangs 14a der Wandung 10 versetzt ist. Vorzugweise fluchtet die Außenfläche 24f des freien Abschnitts 24c mit der Außenkante 15 des Stegs 11.

Die Herstellung der medizinischen Vorrichtung kann durch ein Abtragsverfahren erfolgen. Ein derartiges Abtragsverfahren ist beispielsweise in der auf die Anmelderin zurückgehenden internationalen Patentanmeldung WO 2008/022799 A2 beschreiben und wird hiermit durch Verweis vollständig mit offenbart. Alternativ kann die Stützstruktur aus Stegen 11 und dem Membran 20 separat hergestellt und anschließend miteinander verbunden werden. Die Stützstruktur und die Membran 20 können jeweils flächig hergestellt und miteinander verbunden werden. In einem weiteren Schritt kann die Stützstruktur gemeinsam mit der verbundenen Membran 20 in die Rohrform gebogen werden. Die flexible Membran 20 kann einstückig hergestellt und mit den Stegen 11 verbunden werden. Es ist auch möglich, dass mehrere separat hergesellte Membrane 20 mit einer Stützstruktur aus Stegen 11 verbunden werden. Die Membran 20, insbesondere die Klappen 21 können mit den Stegen 11 verklebt oder verschweißt, insbesondere laser- oder widerstandsverschweißt, werden.

Mögliche Materialien für die Membran 20 sind Kunststoffe, insbesondere Polyurethan. Die flexible Membran 20 umfasst in bevorzugter Weise eine metallische Legierung, insbesondere Nitinol. Die Stege 11 können ebenfalls aus einer metallischen Legierung, vorzugsweise Nitinol, hergestellt sein. Die Stege 11 können beispielsweise durch ein Laserschneidverfahren hergestellt sein. Es ist auch möglich, die Stege 11 durch ein Sputterverfahren, insbesondere ein Sputter-Ätz-Sputter-Verfahren, gebildet werden. Die Stege 11 bzw. die rohrförmige Wandung 10 und die Membran 20 können aus einem rohrförmigen Rohmaterial oder aus einem flächigen Material hergestellt werden. Bei Verwendung eins flächigen Rohmaterials folgt anschließend ein weiterer Verfahrensschritt, bei dem die Rohrform hergestellt wird.

Die Klappen 21 können durch ein Laserschneidverfahren hergestellt werden, wobei die Membran 20 entsprechend strukturiert bzw. geschnitten wird. Die Membran 20, insbesondere die Klappen 21 können auch durch ein Sputterverfahren und/oder ein Ätzverfahren hergestellt werden. Analog zur Herstellung der Stege 11 kann die Membran 20, insbesondere die Klappen 21 aus einem rundförmigen oder einem flachförmigen Rohmaterial hergestellt werden. Beispielsweise kann die Membran 20 aus einem flächigen Material hergestellt, anschließend gerollt und auf eine bereits rohrförmig vorliegende Gitterstruktur aus Stegen 11 aufgebracht werden. Die Membran 20 und die Stege 11 können auch integral hergestellt werden. Geeignete Verfahren hierzu sind beispielsweise Laserabtragsverfahren, Ätzverfahren, insbesondere photochemisches Ätzen, oder Electro Chemical Machining.

Im Hinblick auf weitere bevorzugte Herstellungsverfahren wird auf die gleichzeitig eingereichte deutsche Patentanmeldung "Medizinisches Implantat" verwiesen, die auf die Anmelderin zurückgeht und deren Offenbarungsgehalt vollumfänglich in die vorliegende Anmeldung aufgenommen wird.

Im Rahmen eines weiteren Herstellungsverfahrens für Abdeckungen, die sich mit der Gitterstruktur überlappen, kann die Abdeckung so hergestellt werden, dass sie sich nur in einen Bereich innerhalb einer Zelle erstreckt. Bei diesem ersten Herstellungsschritt kommt es zu keiner Überlappung. Durch die spätere Verformung der Zelle, zum Beispiel durch die Komprimierung der Zelle in Umfangsrichtung, erfolgt die Überlappung der Abdeckung mit der Gitterstruktur. In diesem Zustand wird der Ruhezustand der Zelle wieder fixiert, zum Beispiel durch eine Wärmebehandlung. Die Zelle nimmt ihre endgültige Geometrie im Ruhezustand an. Die Gitterstruktur, bzw. der Stent weist daher im Ruhezustand Abdeckungen auf, die sich an der Gitterstruktur überlappen. Diese Herstellungstechnik ist einfach zu verwirklichen und eignet sich für alle Geometrien, bei denen sich die Abdeckung mit der Gitterstruktur überlappt. Bei einer Überlappung im Ruhezustand wird verhindert, dass sich die Folie, bzw. die Abdeckung beim Crimpen in die Zelle hinein biegt.

Im Hinblick auf die Dimensionen und Materialien der Klappe 21 wird allgemein darauf hingewiesen, dass die zweckmäßige Ventilfunktion sowohl mit einer Auslenkung der Klappe 21 nach radial außen, als auch mit einer Auslenkung der Klappe 21 nach radial innen erreicht wird. Die Klappe 21 ist grundsätzlich biegsam bzw. flexibel, so dass die Klappe 21 wenigstens in eine Richtung radial aus der Wandungsebene der rohrförmigen Wandung 10 auslenkbar bzw. verformbar ist. Die Verformung bzw. Auslenkung kann nach innen und/oder nach außen bezogen auf die rohrförmige Wandung 10 erfolgen. Die Flexibilität bzw. Biegsamkeit oder Auslenkbarkeit der Klappe 21 kann derart angepasst sein, dass unterschiedliche Öffnungsraten bzw. Öffnungsgrade der Klappe 21 einstellbar sind. Die Klappe 21 kann derart angepasst sein, dass eine bevorzugte Öffnungsrichtung eingestellt ist. Die Klappe öffnet sich beispielsweise bei Beaufschlagung mit einem Druckgradienten bevorzugt in eine Vorzugsrichtung, unabhängig davon, in welche Richtung der Druckgradient wirkt. Generell ist das Verhalten der Klappe 21 durch die Anordnung der Klappe 21 im Bezug auf die Stege 11 und die Positionierung der Klappe 21 auf dem Außenumfang oder dem Innenumfang der rohrförmigen Wandung 10 beeinflussbar.

Es ist auch möglich, dass die Klappe 21 derart flexibel und wirksam ist, dass die Klappe 21 die Offenstellung auch dann einnimmt, wenn äußere, auf die Klappe 21 wirkende, Kräfte vernachlässigbar klein sind. Dies ist beispielsweise dann der Fall, wenn die äußeren auf die Klappe 21 wirkenden Kräfte kaum messbar sind. Dabei kann die Flexibilität bzw. Biegsamkeit der Klappe 21 derart angepasst sein, dass die Klappe 21 sich in Richtung der Fluidströmung ausrichtet, wobei eine Rückwirkung der Klappe 21 auf die Fluidströmung vernachlässigbar klein ist. Insbesondere wird dadurch vermieden, dass es durch eine Rückwirkung der Klappe 21 auf die Fluidströmung zu einem Druckabfall kommt. Dabei ist nicht ausgeschlossen, dass ein minimaler, im Wesentlichen kaum messbarer, Druckabfall durch die Reibung des Fluids an der Oberfläche der Klappe 21 entsteht.

Die medizinische Vorrichtung kann durch die flexible Membran 20, die wenigstens eine Klappe 21 bildet, mit der Fluidströmung transportierte Partikel in eine Vorzugsrichtung leiten. Diese Funktion ist insbesondere durch Auslegung der Dimension bzw. der Materialien oder der mechanischen Eigenschaften der Klappe 21 einstellbar. Insbesondere ist der Durchfluss bzw. der Durchflussanteil durch die Zelle 12 durch die Öffnungsrate bzw. den Öffnungsgrad der Klappe 21 einstellbar. Bei kleinen Durchflussraten der Fluidströmung ist es beispielsweise möglich, dass kleine Mengen des Körperfluids, beispielsweise kleine Blutmengen, distal gelegenes Gewebe des Nebengefäßes 42 versorgen. Partikel, insbesondere Thrombenpartikel fließen hingegen mit der Hauptströmung und werden daher nicht durch die Zellen 12 der rohrförmigen Wandung 10 transportiert. Die Wandstärke der Klappe 21 sowie die Geometrie der Klappe 21 sind in diesem Zusammenhang einstellbare Parameter, die das Verhalten der Klappe 21 entsprechend beeinflussen.

Es ist möglich, dass die Öffnungsrate bzw. der Öffnungsgrad in Abhängigkeit der Beanspruchung bzw. der äußeren, auf die Klappe 21 wirkende Kräfte, beispielsweise einem Druckgradienten, veränderbar sind. Dasselbe gilt für die Biegsamkeit bzw. die Flexibilität der Klappe 21, die ebenfalls in Abhängigkeit von äußeren, auf die Klappe 21 wirkende Kräfte veränderbar sein können. Wenn die medizinische Vorrichtung zur Behandlung von Aneurysmen eingesetzt wird, können beispielsweise in einem mittleren Bereich der medizinischen Vorrichtung, insbesondere in dem das Aneurysma abdeckenden Bereich, Klappen 21 vorgesehen sein, die eine relativ hohe Steifigkeit aufweisen, um eine Fluidströmung in das Aneurysma zuverlässig zu blockieren. An den axialen Enden der medizinischen Vorrichtung bzw. der rohrförmigen Wandung 10 können die Klappen 21 hingegen eine erhöhte Flexibilität bzw. Biegsamkeit aufweisen, um den Blutstrom in Nebengefäße 42 zu ermöglichen.

Bei der Behandlung von Stenosen, beispielsweise im Bereich der Halsschlagadern (Karotiden), kann hingegen eine hohe Flexibilität der Klappen 21 im mittleren Bereich der rohrförmigen Wandung 10 vorteilhaft sein. Dadurch weisen die Klappen 21 im mittleren Bereich der rohrförmigen Wandung 10 eine relativ hohe Öffnungsrate bzw. einen relativ hohen Öffnungsgrad auf, so dass die Durchblutung eines Nebengefäßes 42, insbesondere der äußeren oder inneren Halsschlagader (a. carotis externa bzw. a. carotis interna) ermöglicht ist. An den axialen Enden der rohrförmigen Wandung 10 können die Klappen 21 hingegen eine relativ hohe Steifigkeit aufweisen, um die Stenose schützend abzudecken.

Die Flexibilität bzw. Biegsamkeit und/oder weitere Eigenschaften der Klappen 21 können sich generell in Längsrichtung oder Umfangsrichtung der rohrförmigen Wandung 10 ändern. Beispielsweise kann die medizinische Vorrichtung einen Bereich aufweisen, der Klappen 21 mit ersten Klappeneigenschaften aufweist. Radial gegenüberliegend kann ein weiterer Bereich vorgesehen sein, der Klappen 21 mit zweiten Klappeneigenschaften umfasst. Zur Behandlung von Aneurysmen ist es vorteilhaft, wenn die ersten Klappeneigenschaften eine erhöhte Flexibilität bzw. Biegsamkeit der Klappen 21 bewirken und die zweiten Klappeneigenschaften eine erhöhte Steifigkeit der Klappen 21 umfassen. Generell ist es auch möglich, dass die Öffnungsrate bzw. der Öffnungsgrad der Klappen 21 durch die Größe, also die flächige Erstreckung der Klappe 21, einstellbar ist.

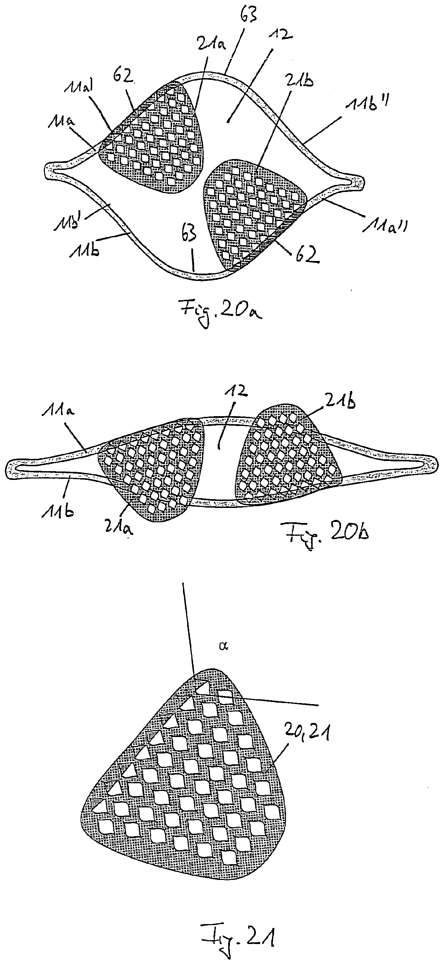

Bei dem Ausführungsbeispiel gemäß Fig. 20a, 20b ist vorgesehen, dass eine Zelle 12 zwei Klappen 21a, 21b aufweist, die in einem expandierten Zustand der Zelle 12 in Gegenüberstellung (Fig. 20a) und in einem komprimierten Zustand der Zelle 12 seitlich nebeneinander angeordnet sind (Fig. 20b). Die Gegenüberstellung der Klappen 21 bedeutet, dass die beiden zweiten freien Enden 22b der Klappen 21a, 21b einander gegenüber angeordnet sind. Die zweiten freien Enden 22b können dabei direkt gegenüber, insbesondere im Wesentlichen fluchtend, oder versetzt zueinander und gegenüber, insbesondere mit versetzten Längsachsen der Klappen 21a, 21b, angeordnet sein. Die zweiten Enden 22b der beiden Klappen 21a, 21b können dabei in etwa auf derselben Höhe oder beabstandet voneinander (Fig. 20a) oder etwas überlappend angeordnet sein. Die zweiten Enden 22b der beiden Klappen 21a, 21b sind vom ersten Ende 22a der jeweils anderen Klappe 21a, 21b beabstandet.

In der seitlich nebeneinander angeordneten Stellung (Fig. 20b) sind die Seiten der Klappen 21a, 21b, die sich zwischen den ersten und zweiten Enden 22a, 22b erstrecken, nebeneinander angeordnet. Die zweiten Enden 22b der beiden Klappen 21a, 21b sind in etwa auf der gleichen Höhe wie das erste Ende 22a der jeweils anderen Klappe 21a, 21b angeordnet. Der Unterschied zwischen dem komprimierten und expandierten Zustand besteht im Allgemeinen darin, dass die zweiten Enden 22b der beiden Klappen 21a, 21b im expandierten Zustand von den ersten Enden 22a der beiden Klappen 21a, 21b in Längsrichtung der Klappen 21a, 21b weiter entfernt angeordnet sind als im komprimierten Zustand. Außerdem sind die zweiten Enden 22b der beiden Klappen 21a, 21b im expandierten Zustand in Längsrichtung einander gegenüber (beabstandet oder überlappend oder auf gleicher Höhe) und im komprimierten Zustand nebeneinander versetzt angeordnet.

Die Klappen 21a, 21b sind jeweils an einem geraden Abschnitt 62 der Stege 11a, 11b befestigt, der sich zwischen den Knotenpunkten und den gekrümmten Abschnitten 63 der Zelle im expandierten Zustand (Fig. 20a) befinden. Die Klappen 21a, 21b sind jeweils an im wesentlichen parallelen Stegabschnitten 11a', 11a" des ersten und zweiten Stegs 11a, 11b vorgesehen. Es ist auch möglich die Klappen 21a, 21b um 90° gedreht an den anderen beiden parallelen Stegabschnitten 11b', 11b" vorzusehen. Mit dieser Anordnung wird die Crimparkeit der Struktur verbessert und außerdem eine große Zellüberdeckung erreicht.

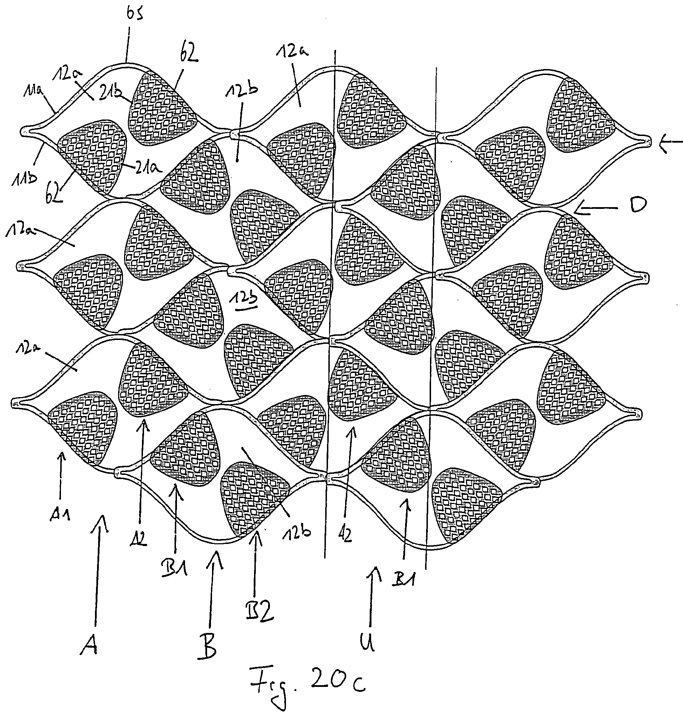

In Fig. 20c ist die Anordnung mehrerer Zellen 12 dargestellt, die gemäß Fig. 20a ausgebildet sind. Wenn, wie in Fig. 20c gezeigt, über den Umfang der Wandung 10 mehrere Klappen 21 vorgesehen sind, ist es bevorzugt, dass die Klappen 21 gleichartig orientiert sind. Die Klappen 21, insbesondere benachbarte Klappen 21 weisen also im Wesentlichen dieselbe Längsorientierung auf. Dabei ist zu konkretisieren, dass die Klappen 21a, 21b einer Zelle 12 unterschiedlich orientiert sind, wie in Fig. 20a, 20c und der zugehörigen Beschreibung offenbart. Daraus ergibt sich folgendes Anordnungsmuster.

Die beiden Klappen 21a, 21b einer Zelle 12 sind in unterschiedlichen Anordnungen, insbesondere in zwei unterschiedlichen Anordnungen vorgesehen. Die beiden Anordnungen unterscheiden sich darin, dass die Klappen 21a, 21b verschiedener Zellen 12a, 12b umgekehrt orientiert sind. Konkret sind die Klappen 21a, 21b der ersten Zellen 12a um 90° gedreht zu den Klappen 21a, 21b der zweiten Zellen 12b angeordnet. Andere Winkel sind möglich.

Wie in Fig. 20c gut zu erkennen, sind in Umfangsrichtung der Gitterstruktur (in Fig. 20c entspricht dies der vertikalen Richtung) die ersten Zellen 12a in einem Segment A und die zweiten Zellen 12b in einem weiteren Segment B angeordnet. Die Segmente A, B bilden jeweils erste Zellreihen, die sich jeweils in Umfangsrichtung der Gitterstruktur erstrecken. Innerhalb der Zellreihen bilden die ersten und zweiten Klappen 21a, 21b wiederum Reihen A1, A2 und B1, B2 in Umfangsrichtung, wobei die ersten Klappen 21a die ersten Reihen A1, B1 und die zweiten Klappen 21b die zweiten Reihen A2, B2 der Segmente A, B bilden. Die Segmente A, B sind in Längsrichtung der Gitterstruktur einander nachgeordnet, insbesondere einander direkt nachgeordnet. Die ersten Zellen 12a des einen Segments A sind in Umfangsrichtung versetzt zu den zweiten Zellen 12b des nächsten Segments B angeordnet.

In Längsrichtung der Gitterstruktur sind Segmente C, D in unterschiedlichen Höhen angeordnet, die zeilenartig ausgebildet sind und jeweils zellenweise ausgerichtete Klappen 21a, 21b aufweisen. Die Anordnung der Klappen 21a, 21b entspricht der Anordnung gemäß Fig. 20a einschließlich der umgekehrt orientierten Klappenanordnung. Die Anordnung der ersten und zweiten Zellen 12a, 12b einschließlich der Klappenanordnung gemäß der Segmente A, B (Umfangsrichtung) entspricht in analoger Weise der Anordnung der ersten und zweiten Zellen 12a, 12b einschließlich der Klappenanordnung gemäß der Segmente C, D (Längsrichtung).

Die Ausrichtung der Klappen 21a, 21b zueinander in einer Zelle 12a, 12b, d.h. die Gegenüberstellung im expandierten Zustand bzw. die Nebeneinanderanordnung im komprimierten Zustand wiederholt sich in den anderen Zellen 12a, 12b ein und desselben Segments A bzw. des Segments B. Insofern ist die Gesamtausrichtung jeweils zweier Klappen 21a, 21b einer Zelle 12 pro Segment A bzw. pro Segment B jeweils gleich. Die Ausrichtung der Klappen 21a, 21b der ersten Zellen 12a des einen Segments A ist anders orientiert bzw. gedreht, insbesondere um 90° gedreht, als die Ausrichtung der Klappen 21a, 21b der zweiten Zellen 12a des in Längsrichtung nächsten Segments B.

Entsprechendes gilt für die sich in Längsrichtung erstreckenden Segmente C, D.

Im komprimierten Zustand und/oder beim Crimpen, d.h. beim Übergang vom expandierten zum komprimierten Zustand überlappen die Klappen 21b der zweiten Reihe A2 des einen Segments A mit den Klappen 21a der ersten Reihe B1 des nächsten Segments derart, dass im komprimierten Zustand die Klappen 21a, 21b der jeweiligen Reihe A2, B1 in die gleiche Richtung, insbesondere in Umfangsrichtung orientiert sind. Es ist auch möglich, dass Klappen im expandierten Zustand teilweise überlappen.

Die Anordnung der Klappen 21b der zweiten Reihe A2 des einen Segments A und der Klappen 21a der ersten Reihe B1 des nächsten Segments B kann sich ferner daraus ergeben, dass die Klappen 21b der zweiten Reihe A2 des einen Segments A und die Klappen 21a der ersten Reihe B1 des nächsten Segments B in dem mit U bezeichneten Bereich in Fig. 20c die gleiche Umfangsrichtung aufweisen.

Bei dem Ausführungsbeispiel gemäß Fig. 20a, 20b wird die Verformung der Klappen 21 durch deren Befestigung an den geraden Stegabschnitten minimiert.

Die Klappen 21a, 21b sind so ausgestaltet, dass diese auf den Stegen gleiten können. Sie sind gleitfähig. Dazu sind die Klappen 21a, 21b auf einer Stegaußen- oder Steginnenseite, also auf einer Ober- oder Unterseite der Stege befestigt. Überdies ist die Wandstärke der Klappen 21a, 21b geringer als die Wandstärke der Stege. Die Wandstärke der Klappen 21a, 21b kann im Verhältnis zur Wandstärke der Stege höchstens 50%, insbesondere höchstens 40%, insbesondere höchstens 30%, insbesondere höchstens 20%, insbesondere höchstens 10%, insbesondere höchstens 5% betragen.

Ein weiterer Vorteil der Klappen 21a, 21b ist deren Einziehbarkeit. Dazu sind die Kanten der Klappen 21a, 21b, insbesondere die in Einziehrichtung vorderen bzw. proximalen Kanten, so ausgebildet, dass diese im komprimierten Zustand der Gitterstruktur gemäß Fig. 20b bezogen auf eine Ebene senkrecht zur Mittellängsachse der Vorrichtung unter einem Winkel geneigt angeordnet sind. Da die Zelle im Beispiel gemäß Fig. 20b so gestreckt ist, dass die Stege fast parallel zur Mittellängsachse verlaufen, sind die Kanten unter einem Winkel von mehr als 90° zu den Stegen 11a, 11b geneigt. Bei anderen Komprimierungsgraden, bei den die Stege weniger stark ausgelenkt werden, wird als Referenz für den Neigungswinkel der Kanten die Ebene senkrecht zur Mittellängsachse der Vorrichtung herangezogen. Der Neigungswinkel bezogen auf die Ebene senkrecht zur Mittellängsachse der Vorrichtung kann mindestens 10°, insbesondere mindestens 15°, insbesondere mindestens 20°, insbesondere mindestens 25°, insbesondere mindestens 30°, insbesondere mindestens 35°, insbesondere mindestens 40°, insbesondere mindestens 45°, insbesondere mindestens 50°, insbesondere mindestens 55°, insbesondere mindestens 60° betragen.

Im expandierten Zustand wird der Kantenwinkel größer und zwar in Abhängigkeit vom Tipwinkel, der den Winkel zwischen den beiden Stegen 11a, 11b am Verbinder beschreibt. Der Tipwinkel beträgt mindestens 60°, insbesondere mindestens 80°, insbesondere mindestens 90°, insbesondere mindestens 100°, insbesondere mindestens 120°.

Bei dem Ausführungsbeispiel gemäß Fig. 20b überlappen die Klappen 21a, 21b jeweils eine einzige Nachbarzelle. Es ist möglich, dass die eine Klappe 21a und/oder die andere Klappen 21b jeweils mindestens eine Nachbarzelle, insbesondere mindestens zwei Nachbarzellen, insbesondere mindestens drei Nachbarzellen, insbesondere mindestens vier Nachbarzellen, insbesondere mindestens fünf Nachbarzellen, insbesondere mindestens sechs Nachbarzellen überlappen.

Bei dem Ausführungsbeispiel gemäß Fig. 21 ist eine dazu alternative oder zusätzliche Maßnahme beschrieben, die einer Vermeidung oder Verringerung der Abdeckungsverformung dient und die Anbringung der Klappen auch an gekrümmten Abschnitten ermöglicht. Die Struktur der Abdeckung bzw. der Klappe, insbesondere die Perforierung erstreckt sich bis zum Rand des Steges 11a, an dem die Klappe 21 befestigt ist. Die Verbindung der Folie bzw. Klappe 21 mit dem Steg 11a ist an mehreren Stellen unterbrochen. Bei der Streckung des Steges 11a beim Crimpen, ebenso wie bei der Krümmung bei der Expansion verformen sich die Öffnungen der Strukturierung bzw. der Porenstruktur 28 im Stegbereich, so dass sich die Folie bzw. Klappe nicht oder nur unwesentlich verformt. Insbesondere ändert sich der Winkel α der Zellen der Perforierung oder die Form der Schlitze. Der Winkel α sollte möglichst groß sein, und zwar wie in der übrigen Strukturierung, insbesondere wenigstens 90°, insbesondere wenigstens 120°, insbesondere wenigstens 130°, insbesondere wenigstens 140°, insbesondere wenigstens 150°, insbesondere wenigstens 160°, insbesondere wenigstens 170°. Die Zellen bzw. Öffnungen der Perforierung sind vorzugsweise mit dem Verlauf des Steges 11a ausgerichtet.

Vorteilhaft ist vorgesehen, dass die Klappen 21 einen Kunststoff, insbesondere Polyurethan, umfassen. Besonders bevorzugt ist es, wenn die Klappen 21 eine Nickel-Titan-Legierung, insbesondere Nitinol, aufweisen. Eine Kombination aus einem Kunststoff und einer Nickel-Titan-Legierung ist möglich. Beispielsweise können entsprechende Kompositwerkstoffe eingesetzt werden. Weitere mögliche Materialien umfassen biodegradierbare Werkstoffe, beispielsweise Magnesium oder Magnesiumlegierungen. Die Auflösung der Klappen kann in bestimmten Fällen gewünscht sein und beschleunigt die Endothelialisierung, wenn die akute Funktion der Klappen nicht mehr gebraucht wird. Die ganze Struktur oder auch nur die Klappen können teilweise oder vollständig biodegradierbar sein. Es ist auch möglich, dass Medikamente (Gentherapeutika, Antikoagulanzien oder andere Stoffe) innerhalb des biodegradierbaren Materials, oder an dessen Oberfläche, oder auch in Mikro- oder Nanostrukturen im Material eingelagert sind.

Die medizinische Vorrichtung kann mit dem folgenden Verfahren hergestellt werden, das die Schritte umfasst:
- Bereitstellen einer Rohform der Gitterstruktur;
- Aufbringen einer Opferschicht umfassend einen Fotolack und/oder eine Opferfolie auf die Gitterstruktur;
- Strukturieren der Opferschicht, wobei eine Struktur von Verbindungsstellen der Gitterstruktur, insbesondere Stegabschnitte freigelegt wird;
- Aufbringen einer Abdeckschicht auf die Opferschicht und die Verbindungsstellen derart, dass die Abdeckschicht an den Verbindungsstellen mit der Gitterstruktur verbunden wird und ein Abdeckelement, insbesondere eine Membran bzw. Klappe bildet; und
- Entfernen der Opferschicht zur Bildung des Abdeckelements.

Zu den Einzelheiten des Verfahrens wird auf die am selben Tag eingereichte Anmeldung "Medizinisches Implantat und Verfahren zum Herstellen eines derartigen Implantats" verwiesen, die auf die Anmelderin zurückgeht und die vollständig in den Offenbarungsgehalt dieser Anmeldung eingeschlossen wird.

Andere bekannte Herstellungsverfahren sind auch möglich, bspw. die Verbindung der Klappen mit den Stegen durch Laserschweißen.

### Bezugszeichenliste

- 10: Wandung
- 11: Steg
- 11a: erster Steg
- 11b: zweiter Steg
- 11a', 11a": Stegabschnitte
- 11b', 11b": Stegabschnitte
- 12: Zelle
- 12a: erste Reihe
- 12b: zweite Reihe
- 13: Knotenpunkt
- 13a: erster Knotenpunkt
- 13b: zweiter Knotenpunkt
- 14a: Außenumfang
- 14b: Innenumfang
- 15: Außenkante
- 20: Membran
- 21: Klappe
- 21a: erste Klappe
- 21b: zweite Klappe
- 22a: erstes Ende
- 22b: zweites Ende
- 22c: freie Kante
- 22d: Halteabschnitt
- 22e: innere Kante
- 23: Faltlinie
- 23a: Nut
- 23b: Spalt
- 23c: Spaltkante
- 24a: erster Klappenflügel
- 24b: zweiter Klappenflügel
- 24c: freier Abschnitt
- 24d: fixierter Abschnitt
- 24e: Außenfläche
- 25: Mehrfachklappe
- 26: Krümmung
- 27: Spitze
- 28: Porenstruktur
- 29: Faltöffnung
- 30: axialer Hohlkanal
- 40: Gefäßwand
- 41: Hauptgefäß
- 42: Nebengefäß
- 43: Partikel
- 50: Aneurysma
- 51: Stenose
- 60: Katheter
- 61: Coil
- 62: gerader Abschnitt
- 63: gekrümmter Abschnitt

## Patentansprüche

1. Medizinische Vorrichtung mit einer rohrförmigen Wandung (10) aus Stegen (11), die Zellen (12) begrenzen, und einer flexiblen Membran (20), die wenigstens eine Klappe (21) bildet, die
- ein erstes Ende (22a), das mit wenigstens einem ersten Steg (11a) einer Zelle (12) verbunden ist, und
- ein freies zweites Ende (22b) aufweist, das dem ersten Ende (22a) in Längsrichtung der Klappe (21) gegenüber angeordnet ist,
und die Klappe (21)
- in eine Schließstellung, in der sich die Klappe (21) entlang der rohrförmigen Wandung (10) erstreckt und die Zelle (12) zumindest teilweise verschließt, und
- in eine Offenstellung selbsttätig in Folge eines Druckgradienten oder einer Fluidströmung überführbar ist, in der die Klappe (21) relativ zur Wandung (10) radial ausgelenkt ist, um die Zelle (12) ventilartig freizugeben,
**dadurch gekennzeichnet, dass**
die Klappe (21) mit dem Steg (11a) linienförmig verbunden ist.

2. Medizinische Vorrichtung, insbesondere Stent, mit einer rohrförmigen Wandung (10), die einen axialen Hohlkanal (30) bildet und Stege (11) aufweist, die Zellen (12) begrenzen, wobei wenigstens zwei flexible Membrane (20) vorgesehen sind, die in Längsrichtung des axialen Hohlkanals (30) voneinander beabstandet angeordnet sind und jeweils wenigstens eine Klappe (21) bilden, die
- ein erstes Ende (22a), das mit wenigstens einem ersten Steg (11a) einer Zelle (12) verbunden ist, und
- ein freies zweites Ende (22b) aufweist, das dem ersten Ende (22a) in Längsrichtung der Klappe (21) gegenüber angeordnet ist,
und die Klappe (21)
- in eine Offenstellung, in der sich die Klappe (21) entlang der rohrförmigen Wandung (10) erstreckt, und
- in eine Schließstellung selbsttätig in Folge eines Druckgradienten oder einer Fluidströmung überführbar ist, in der die Klappe (21) relativ zur Wandung (10) radial ausgelenkt ist und in den axialen Hohlkanal (30) hineinragt, um den axialen Hohlkanal (30) ventilartig zumindest teilweise zu verschließen,
**dadurch gekennzeichnet, dass**
die Klappe (21) mit dem Steg (11a) linienförmig verbunden ist.

3. Medizinische Vorrichtung nach Anspruch 1 oder 2,
**dadurch gekennzeichnet, dass**
die Zelle (12) wenigstens einen ersten Knotenpunkt (13) aufweist, der den ersten Steg (11a) mit einem zweiten Steg (11b) verbindet, wobei der erste Steg (11a) und der zweite Steg (11b) im Bereich des Knotenpunktes (13) einen Winkel einschließen und das erste Ende (22a) der Klappe (21) in der Nähe, insbesondere im Bereich, des Knotenpunktes (13) mit dem ersten Steg (11a) und/oder dem zweiten Steg (11b) verbunden ist, und/oder das erste Ende (22a) der Klappe (21) auf einem Außenumfang (14a) oder einem Innenumfang (14b) der rohrförmigen Wandung (10) angeordnet ist, und/oder die Klappe (21) eine im Wesentlichen blattartige Form aufweist, und/oder die Klappe (21) eine Oberflächenstrukturierung, insbesondere eine Porenstruktur (28) oder eine Rillenstruktur oder eine Fleecestruktur, aufweist.

4. Medizinische Vorrichtung nach wenigstens einem der vorhergehenden Ansprüche,
**dadurch gekennzeichnet, dass**
die Klappe (21) eine Faltlinie (23) aufweist, die sich zumindest abschnittsweise von dem ersten Ende (22a) zum freien, zweiten Ende (22b) erstreckt, und die Faltlinie (23) die Klappe in einen ersten Klappenflügel (24a) und einen zweiten Klappenflügel (24b) unterteilt, und der erste Klappenflügel (24a) mit dem ersten Steg (11a) der Zelle (12) und der zweite Klappenflügel (24b) mit dem zweiten Steg (11b) der Zelle (12) verbunden ist.

5. Medizinische Vorrichtung nach Anspruch 4,
**dadurch gekennzeichnet, dass**
die Faltlinie (23) eine Nut (23a) und/oder einen Spalt (23b) umfasst.

6. Medizinische Vorrichtung nach Anspruch 5,
**dadurch gekennzeichnet, dass**
der Spalt (23b) den ersten Klappenflügel (24a) zumindest abschnittsweise von dem zweiten Klappenflügel (24b) trennt.

7. Medizinische Vorrichtung nach Anspruch 5,
**dadurch gekennzeichnet, dass**
der Spalt (23b) den ersten Klappenflügel (24a) vollständig von dem zweiten Klappenflügel (24b) trennt derart, dass der erste Klappenflügel (24a) von dem zweiten Klappenflügel (24b) beabstandet angeordnet ist.

8. Medizinische Vorrichtung nach wenigstens einem der Ansprüche 1 bis 7,
**dadurch gekennzeichnet, dass**
die Klappe (21) selbsttätig von der Schließstellung in die Offenstellung überführbar ist.

9. Medizinische Vorrichtung nach wenigstens einem der Ansprüche 1 oder 3 bis 8,
**dadurch gekennzeichnet, dass**
das freie zweite Ende (22b) der Klappe (21) in der Schließstellung in die Zelle (12) hineinragt, insbesondere innerhalb der Zelle (12) angeordnet ist, oder die Zelle (12) überlappt.

10. Medizinische Vorrichtung nach wenigstens einem der Ansprüche 1 oder 3 bis 9,
**dadurch gekennzeichnet, dass**
sich die Klappe (21) in der Schließstellung in Längsrichtung oder in Umfangsrichtung der rohrförmigen Wandung (10) erstreckt.

11. Medizinische Vorrichtung nach wenigstens einem der Ansprüche 1 oder 3 bis 10,
**dadurch gekennzeichnet, dass**
die Membran (20) mehrere Klappen (21) aufweist, die sich in der Schließstellung entlang der rohrförmigen Wandung (10) erstrecken.

12. Medizinische Vorrichtung nach Anspruch 11,
**dadurch gekennzeichnet, dass**
jeweils ein freies zweites Ende (22b) einer ersten Klappe (21a) zumindest im komprimierten Zustand der rohrförmigen Wandung wenigstens ein mit einem Steg (11) verbundenes, erstes Ende (22a) einer zweiten Klappe (21b) dachziegelartig überlappt.

13. Medizinische Vorrichtung nach wenigstens einem Ansprüche 2 bis 7,
**dadurch gekennzeichnet, dass**
die wenigstens zwei Membrane (20) jeweils eine Mehrfachklappe (25) bilden, die von einer Schließstellung in eine Offenstellung überführbar ist, wobei die Mehrfachklappe (25) wenigstens zwei Klappen (21) aufweist, die bezogen auf eine Längsachse des axialen Hohlraums (30) radial gegenüber angeordnet sind und sich in der Schließstellung in den axialen Hohlraum (30) erstrecken, um den axialen Hohlraum (30) ventilartig zu verschließen.

14. Medizinische Vorrichtung nach Anspruch 13,
**dadurch gekennzeichnet, dass**
sich die freien, zweiten Enden (22b) der Klappen (21) in der Schließstellung berühren.

15. Medizinische Vorrichtung nach wenigstens einem Ansprüche 1 bis 14,
**dadurch gekennzeichnet, dass**
eine Zelle (12) zwei Klappen (21) aufweist, die in einem expandierten Zustand der Zelle (12) in Gegenüberstellung und in einem komprimierten Zustand der Zelle (12) seitlich nebeneinander angeordnet sind, und/oder zumindest im Bereich des ersten Endes (22a) der Klappe (21) eine Porenstruktur (28), insbesondere eine Perforierung der Klappe (21) ausgebildet ist, die sich bis in den Bereich des ersten Steges (11a) erstreckt, wobei das erste Ende (22a) abschnittsweise mit dem ersten Steg (11a) verbunden ist derart, dass die Porenstruktur (28) des ersten Endes (22a) im losen Bereich oder in den losen Bereichen verformbar ist.

## Claims

1. A medical device with a tubular wall (10) produced from webs (11) which delimit cells (12), and with a flexible membrane (20) which forms at least one flap (21) which has
- a first end (22a) connected to at least one first web (11a) of a cell (12), and
- a free second end (22b) disposed opposite the first end (22a) in the longitudinal direction of the flap (21),
and wherein the flap (21) can be moved
- into a closed position in which the flap (21) extends along the tubular wall (10) and at least partially closes the cell (12), and
- automatically into an open position as a consequence of a pressure gradient or a fluid flow, in which the flap (21) is radially deflected relative to the wall (10) in order to open the cell (12) in the manner of a valve,
**characterized in that**
the flap (21) is connected to the web (11a) in a linear manner.

2. A medical device, in particular a stent, with a tubular wall (10) which forms an axial hollow channel (30) and has webs (11) which delimit cells (12), wherein at least two flexible membranes (20) are provided which are disposed spaced apart from each other in the longitudinal direction of the axial hollow channel (30) and which each form at least one flap (21) which has
- a first end (22a) connected to at least one first web (11a) of a cell (12), and
- a free second end (22b) disposed opposite the first end (22a) in the longitudinal direction of the flap (21),
and wherein the flap (21) can be moved
- into an open position in which the flap (21) extends along the tubular wall (10), and
- automatically into a closed position as a consequence of a pressure gradient or a fluid flow, in which the flap (21) is radially deflected relative to the wall (10) and protrudes into the axial hollow channel (30) in order to at least partially close the axial hollow channel (30) in the manner of a valve
**characterized in that**
the flap (21) is connected to the web (11a) in a linear manner.

3. The medical device as claimed in claim 1 or claim 2,
**characterized in that**
the cell (12) has at least one first nodal point (13) which connects the first web (11a) to a second web (11b), wherein the first web (11a) and the second web (11b) enclose an angle in the region of the nodal point (13), and the first end (22a) of the flap (21) is connected to the first web (11a) and/or the second web (11b) in the vicinity, in particular in the region, of the nodal point (13), and/or the first end (22a) of the flap (21) is disposed on an outer circumference (14a) or an inner circumference (14b) of the tubular wall (10), and/or the flap (21) is substantially leaf-shaped, and/or the flap (21) has a surface structure, in particular a pore structure (28) or a fluted structure or a fleece structure.

4. The medical device as claimed in at least one of the preceding claims,
**characterized in that**
the flap (21) has a fold line (23) which extends at least in sections from the first end (22a) to the free second end (22b), and the fold line (23) divides the flap into a first flap wing (24a) and a second flap wing (24b) and the first flap wing (24a) is connected to the first web (11a) of the cell (12), and the second flap wing (24b) is connected to the second web (11b) of the cell (12).

5. The medical device as claimed in claim 4,
**characterized in that**
the fold line (23) comprises a groove (23a) and/or a gap (23b).

6. The medical device as claimed in claim 5,
**characterized in that**
the gap (23b) at least partially separates the first flap wing (24a) from the second flap wing (24b).

7. The medical device as claimed in claim 5,
**characterized in that**
the gap (23b) completely separates the first flap wing (24a) from the second flap wing (24b) in a manner such that the first flap wing (24a) is disposed spaced apart from the second flap wing (24b).

8. The medical device as claimed in at least one of claims 1 to 7,
**characterized in that**
the flap (21) can be moved automatically from the closed position into the open position.

9. The medical device as claimed in at least one of claims 1 or 3 to 8,
**characterized in that**
in the closed position, the free second end (22b) of the flap (21) protrudes into the cell (12), in particular is disposed inside the cell (12), or overlaps the cell (12).

10. The medical device as claimed in at least one of claims 1 or 3 to 9,
**characterized in that**
in the closed position, the flap (21) extends in the longitudinal direction or in the circumferential direction of the tubular wall (10).

11. The medical device as claimed in at least one of claims 1 or 3 to 10,
**characterized in that**
the membrane (20) has a plurality of flaps (21) which extend along the tubular wall (10) in the closed position.

12. The medical device as claimed in claim 11,
**characterized in that**
at least in the compressed state of the tubular wall, in each case a free second end (22b) of a first flap (21a) overlaps at least one first end (22a) of a second flap (21b) connected to a web (11) in the manner of a roof tile.

13. The medical device as claimed in at least one of claims 2 to 7,
**characterized in that**
the at least two membranes (20) each form a multiple flap (25) which can be moved from a closed position into an open position, wherein the multiple flap (25) has at least two flaps (21) which are radially oppositely disposed with respect to a longitudinal axis of the axial hollow space (30) and, in the closed position, extend into the axial hollow space (30) in order to close the axial hollow space (30) in the manner of a valve.

14. The medical device as claimed in claim 13,
**characterized in that**
the free second ends (22b) of the flap (21) touch in the closed position.

15. The medical device as claimed in at least one of claims 1 to 14,
**characterized in that**
a cell (12) has two flaps (21) which, in an expanded state of the cell (12), are disposed opposite each other and which, in a compressed state of the cell (12), are disposed laterally alongside each other, and/or at least in the region of the first end (22a) of the flap (21), a pore structure (28) is formed, in particular a perforation of the flap (21), which extends into the region of the first web (11a), wherein a section of the first end (22a) is connected to the first web (11a) in a manner such that the pore structure (28) of the first end (22a) is deformable in the loose region or in the loose regions.

## Revendications

1. Dispositif médical comportant une paroi de forme tubulaire (10) formée de traverses (11), qui limitent les cellules (12), et d'une membrane flexible (20) qui constituent au moins un volet (21), qui présente
- une première extrémité (22a) qui est connecté à au moins une traverse (11a) d'une cellule (12), et
- une seconde extrémité libre (22b) qui est disposée à l'opposé de la première extrémité (22a) dans le sens longitudinal du volet (21),
et le volet (21) peut être passé
- dans une position de fermeture, dans laquelle le volet (21) s'étend le long de la paroi de forme tubulaire (10) et ferme au moins partiellement la cellule (12), et
- automatiquement dans une position d'ouverture suite à un gradient de pression ou à un écoulement de fluide dans laquelle le volet (21) est dévié radialement par rapport à la paroi (10) afin de libérer la cellule (12) à la manière d'une soupape,
**caractérisé en ce que**
le volet (21) est connecté linéairement à la traverse (11a).

2. Dispositif médical, en particulier stent, comportant une paroi de forme tubulaire (10) qui constitue un canal creux axial (30) et présente des traverses (11) qui limitent des cellules (12), étant prévues au moins deux membranes flexibles (20) qui sont disposées éloignées l'une de l'autre dans le sens longitudinal du canal creux axial (30) et constituent respectivement au moins un volet (21) qui présente
- une première extrémité (22a) qui est connectée à au moins une traverse (11a) d'une cellule (12), et
- une seconde extrémité libre (22b) qui est disposée à l'opposé de la première extrémité (22a) dans le sens longitudinal du volet (21),
et le volet (21) peut être passé
- dans une position d'ouverture, dans laquelle le volet (21) s'étend le long de la paroi de forme tubulaire (10), et
- automatiquement dans une position de fermeture suite à un gradient de pression ou à un écoulement de fluide, dans laquelle le volet (21) est dévié radialement par rapport à la paroi (10) et dépasse dans le canal creux axial (30) pour fermer au moins partiellement le canal creux axial (30) à la manière d'une soupape,
**caractérisé en ce que**
le volet (21) est connecté linéairement à la traverse (11a).

3. Dispositif médical selon la revendication 1 ou 2,
**caractérisé en ce que**
la cellule (12) présente au moins un premier point nodal (13) qui connecte la première traverse (11a) à une seconde traverse (11b), la première traverse (11a) et la seconde traverse (11b) circonscrivant un angle au niveau du point nodal (13) et la première extrémité (22a) du volet (21) étant connectée à proximité, en particulier au niveau, du point nodal (13), à la première traverse (11a) et/ou à la seconde traverse (11b), et/ou la première extrémité (22a) du volet (21) étant disposée sur une circonférence extérieure (14a) ou une circonférence intérieure (14b) de la paroi de forme tubulaire (10), et/ou le volet (21) présente une forme ressemblant sensiblement à une feuille et/ou le volet (21) présente une structuration de surface, en particulier une structure poreuse (28) ou une structure rainurée ou une structure velue.

4. Dispositif médical selon au moins une des revendications précédentes,
**caractérisé en ce que**
le volet (21) présente une ligne de pliage (23) qui s'étend du moins par sections de la première extrémité (22a) à la seconde extrémité libre (22b) et la ligne de pliage (23) divise le volet en une première aile de volet (24a) et une seconde aile de volet (24b), et la première aile de volet (24a) est connectée à la première traverse (11a) de la cellule (12) et la seconde aile de volet (24b) à la seconde traverse (11b) de la cellule (12) .

5. Dispositif médical selon la revendication 4,
**caractérisé en ce que**
la ligne de pliage (23) comprend une rainure (23a) et/ou une fente (23b).

6. Dispositif médical selon la revendication 5,
**caractérisé en ce que**
la fente (23b) sépare la première aile de volet (24a) au moins par sections de la seconde aile de volet (24b).

7. Dispositif médical selon la revendication 5,
**caractérisé en ce que**
la fente (23b) sépare totalement la première aile de volet (24a) de la seconde aile de volet (24b) de manière à ce que la première aile de volet (24a) soit disposée à distance de la seconde aile de volet (24b).

8. Dispositif médical selon au moins une des revendications 1 à 7,
**caractérisé en ce que**
le volet (21) peut passer automatiquement de la position de fermeture à la position d'ouverture.

9. Dispositif médical selon au moins une des revendications 1 ou 3 à 8,
**caractérisé en ce que**
la seconde extrémité libre (22b) du volet (21), en position de fermeture, dépasse dans la cellule (12), en particulier est disposée à l'intérieur de la cellule (12) ou recouvre la cellule (12).

10. Dispositif médical selon au moins une des revendications 1 ou 3 à 9,
**caractérisé en ce que**
le volet (21), dans la position de fermeture, s'étend dans le sens longitudinal ou dans le sens circonférentiel de la paroi de forme tubulaire (10).

11. Dispositif médical selon au moins une des revendications 1 ou 3 à 10,
**caractérisé en ce que**
la membrane (20) présente plusieurs volets (21) qui s'étendent, en position de fermeture, le long de la paroi de forme tubulaire (10).

12. Dispositif médical selon la revendication 11,
**caractérisé en ce que**
respectivement une seconde extrémité libre (22b) d'un premier volet (21a), du moins en état comprimé de la paroi de forme tubulaire, recouvre au moins une première extrémité (22a) connectée à une traverse (11) d'un second volet (21b) à la manière d'une tuile de toit.

13. Dispositif médical selon au moins une des revendications 2 à 7,
**caractérisé en ce que** les au moins deux membranes (20) constituent respectivement un volet multiple (25) qui peut être passé d'une position de fermeture à une position d'ouverture, le volet multiple (25) présentant au moins deux volets (21) qui, par rapport à un axe longitudinal de la cavité axiale (30), sont disposés radialement opposés et s'étendent, en position de fermeture, dans la cavité axiale (30) afin de fermer la cavité axiale (30) à la manière d'une soupape.

14. Dispositif médical selon la revendication 13,
**caractérisé en ce que**
les secondes extrémités libres (22b) des volets (21) se touchent en position de fermeture.

15. Dispositif médical selon au moins une des revendications 1 à 14,
**caractérisé en ce**
**qu'**une cellule (12) présente deux volets (21) qui, dans un état dilaté de la cellule (12), sont disposés en position opposée et juxtaposés latéralement dans un état comprimé de la cellule (12), et/ou que, au moins au niveau de la première extrémité (22a) du volet (21), est constituée une structure poreuse (28), en particulier une perforation du volet (21), qui s'étend jusque dans la zone de la première traverse (11a), la première extrémité (22a) étant connectée par sections à la première traverse (11a) de manière à ce que la structure poreuse (28) de la première extrémité (22a) soit déformable dans la zone lâche ou dans les zones lâches.
